# EUROPEAN PATENT APPLICATION

(11) **EP 4 428 158 A1**
(43) Date of publication of application: **11.09.2024**
(21) Application number: 23161180.7
(22) Date of filing: 10.03.2023
(51) Int. Cl.: C07K 16/30

(54) **LUNG CANCER TARGETING HUMAN ANTIBODIES AND THERAPEUTIC USES THEREOF**

(71) Applicant: Istituto Romagnolo per lo Studio dei Tumori "Dino Amadori" - IRST S.r.l., 47014 Meldola (FC) (IT)
(72) Inventor: Mazza, Massimiliano, Meldola (FC) (IT); Nicolini, Fabio, Meldola (FC) (IT); Gaimari, Anna, Meldola (FC) (IT); Mazzotti, Lucia, Meldola (FC) (IT); De Lucia, Anna, Meldola (FC) (IT)
(74) Representative: Pace Napoleone, Maria

(57) **Abstract**

The present invention relates to antibodies or antigen binding fragments thereof targeting in particular lung cancer and to pharmaceutical compositions containing said antibodies, and uses thereof.

## Description

### FIELD OF INVENTION

The present invention relates to human antibodies or antigen binding fragments thereof endowed with lung cancer targeting activity, to pharmaceutical compositions containing said antibodies, and uses thereof.

### BACKGROUND OF THE INVENTION

Tertiary lymphoid structures (TLSs) are organized cellular compartments where B, T and dendritic cells (DCs) harbor to elicit a local immune response [1]. TLS presence and B cell infiltration have emerged as a positive prognostic factor of response to immune checkpoint inhibitors (ICIs) in NSCLC patients [2-7]. B cells especially seem to play a crucial role in sustaining anti-tumor immune responses in multiple ways that are understudied and not fully elucidated in the context of NSCLC. However, there are two possibilities of B cell contribution: antibodies produced in situ might contribute either to antibody-dependent cellular cytotoxicity (ADCC) or to the presentation of intracellular antigens collected from dying tumor cells to other immune cells (i.e. T cells, DCs) [5-7].

Antibodies are useful biomolecules that find application both as diagnostics, as therapeutics or as building blocks to construct specific interventional strategies (e.g. bi-specific T-cell engagers (BiTEs), chimeric antigen receptor (CAR) etc.). Antibodies are stable, their production is standardized and they find several applications in oncology, either as markers to diagnose and monitor disease progression or as therapeutics.

Nowadays immunotherapies have limited efficacy. In particular, the use of very few targets at once per pathology, the identification of novel targets and their testing in a therapeutic setting are actual challenges in academia and the private sector.

The possibility to identify biomolecules endowed with cancer targeting potential, fully biocompatible in humans and reformatted to design novel immunotherapies (i.e. BiTEs, CAR) present a great potential for industrial development [8].

Immunotherapy *per* se has already significantly improved lung cancer outcome, however there is still a considerable fraction of patients that do not respond to those treatments and would potentially benefit from specific adoptive cell therapies or similar immunotherapies. There is still the need of an immunotherapy for lung cancer, in particular for an immunotherapy compatible with the human immune response.

### SUMMARY OF THE INVENTION

Inventors investigated the clonal composition of tumor-infiltrating B lymphocytes by BCR repertoire analysis in NSCLC patients before treatment with ICIs (i.e. PD-1 and PD-L1 inhibitors, such as nivolumab) and identified antibodies produced by such B cells endowed with the ability to target lung tumor surface antigens, in order to drive an antitumor immune response.

Disclosed herein are 3 human antibodies named AB3, AB4 and AB23, identified among the initial pool of 42 candidate antibodies, reconstructed from the most enriched VH and VL sequences, detected in situ from the analysis of the BCR repertoire at the time of diagnosis of NSCLC patients, after the treatment with the ICI nivolumab. The B clones encoding for the identified antibodies were highly enriched in frequency in patients responding to ICI therapy, herein called responder NSCLC patients or responders.

Identified antibodies showed the ability to bind to several lung cancer cell lines both by cytometry and immunohistochemistry (IHC).

These antibodies can advantageously be used in immunotherapy based strategies targeting lung cancer cells, such as ADC, CAR harboring cells, nude antibodies for ADCC, with the advantage that such antibodies are derived from humans and therefore they are compatible with the human immune response and they contribute to ICI induced clinical response in lung cancer patients.

It is therefore an object of the invention an isolated antibody or antigen binding fragment thereof comprising:
a. a heavy chain variable domain (VH) comprising:
   i. a CDR1 sequence of an amino acid sequence of: SEQ ID NO.1;
   ii. a CDR2 sequence of an amino acid sequence of SEQ ID NO. 2; and
   iii. a CDR3 sequence of an amino acid sequence selected from the group consisting of SEQ ID NO. 4, 3 and 5 and/or
b. a light chain variable domain (VL) comprising:
   i. a CDR1 sequence of an amino acid sequence selected from the group consisting of: SEQ ID NO. 9, 6 and 12;
   ii. a CDR2 sequence of an amino acid sequence selected from the group consisting of: SEQ ID NO. 10, 7 and 13; and
   iii. a CDR3 sequence of an amino acid sequence selected from the group consisting of: SEQ ID NO. 11, 8 and 14.

Preferably the isolated antibody or antigen binding fragment thereof comprises as CDRs:
- SEQ ID NO: 1 and SEQ ID NO: 2 and SEQ ID NO: 4 and SEQ ID NO: 9 and SEQ ID NO: 10 and SEQ ID NO: 11 or respective Kabat, IMGT, Chothia, AbM, or Contact CDRs, or
- SEQ ID NO: 1 and SEQ ID NO: 2 and SEQ ID NO: 3 and SEQ ID NO: 6 and SEQ ID NO: 7 and SEQ ID NO: 8 or respective Kabat, IMGT, Chothia, AbM, or Contact CDRs, or
- SEQ ID NO: 1 and SEQ ID NO: 2 and SEQ ID NO: 5 and SEQ ID NO: 12 and SEQ ID NO: 13 and SEQ ID NO: 14 or respective Kabat, IMGT, Chothia, AbM, or Contact CDRs.

In a preferred embodiment, said antibody or antigen binding fragment thereof comprises a heavy chain variable region comprising complementarity determining regions CDR1 comprising the amino acid sequence of SEQ ID N.1, CDR2 comprising the amino acid sequence of SEQ ID N.2 and CDR3 comprising the amino acid sequence of SEQ ID N.4 and a light chain variable region comprising complementarity determining regions CDR1 comprising the amino acid sequence of SEQ ID N.9, CDR2 comprising the amino acid sequence of SEQ ID N.10 and CDR3 comprising the amino acid sequence of SEQ ID N.11. Each of said CDRs may also vary of 1 or 2 amino acids as far as the antibody activity is retained.

In a preferred embodiment, said antibody or antigen binding fragment thereof comprises a heavy chain variable region comprising complementarity determining regions CDR1 comprising the amino acid sequence of SEQ ID N.1, CDR2 comprising the amino acid sequence of SEQ ID N.2 and CDR3 comprising the amino acid sequence of SEQ ID N.3 and a light chain variable region comprising complementarity determining regions CDR1 comprising the amino acid sequence of SEQ ID N.6, CDR2 comprising the amino acid sequence of SEQ ID N.7 and CDR3 comprising the amino acid sequence of SEQ ID N.8. Each of said CDRs may also vary of 1 or 2 amino acids as far as the antibody activity is retained.

In a preferred embodiment, said antibody or antigen binding fragment thereof comprises a heavy chain variable region comprising complementarity determining regions CDR1 comprising the amino acid sequence of SEQ ID N.1, CDR2 comprising the amino acid sequence of SEQ ID N.2 and CDR3 comprising the amino acid sequence of SEQ ID N.5 and a light chain variable region comprising complementarity determining regions CDR1 comprising the amino acid sequence of SEQ ID N.12, CDR2 comprising the amino acid sequence of SEQ ID N.13 and CDR3 comprising the amino acid sequence of SEQ ID N.14. Each of said CDRs may also vary of 1 or 2 amino acids as far as the antibody activity is retained.

Preferably the isolated antibody or antigen binding fragment thereof comprises the CDRs as indicated in anyone of Tables 1-14, preferably in Tables 1 and 2.

Preferably said antibody or antigen binding fragment thereof binds to lung cancer cells. Preferably the isolated antibody or antigen binding fragment thereof comprises:
a. a heavy chain variable domain sequence of an amino acid sequence selected from the group consisting of: SEQ ID NO: 91, SEQ ID NO: 90 and SEQ ID NO: 92; or
b. a light chain variable domain sequence of an amino acid sequence selected from the group consisting of: SEQ ID NO: 94, SEQ ID NO:93 and SEQ ID NO:95; or
c. the heavy chain variable domain of (a) and the light chain variable domain of (b).

Preferably the isolated antibody is AB4 comprising a heavy chain comprising or consisting of a sequence of SEQ ID NO: 91 and a light chain comprising or consisting of a sequence of SEQ ID NO:94, or AB3 comprising a heavy chain comprising or consisting of a sequence of SEQ ID NO: 90 and a light chain comprising or consisting of a sequence of SEQ ID NO:93, or AB23 comprising a heavy chain comprising or consisting of a sequence of SEQ ID NO: 92 and a light chain comprising or consisting of a sequence of SEQ ID NO:95.

Preferably, the isolated antibody comprises a heavy chain encoded by a nucleotide sequence selected from the group consisting of: SEQ ID NO: 97, SEQ ID NO: 96, and SEQ ID NO:98; and a light chain encoded by a nucleotide sequence of selected from the group consisting of: SEQ ID NO:100, SEQ ID NO: 99, and SEQ ID NO:101.

Preferably, the isolated antibody is AB4 and it comprises a heavy chain encoded by a nucleotide sequence of SEQ ID NO: 97 and a light chain encoded by a nucleotide sequence of SEQ ID NO: 100; or is AB3 and it comprises a heavy chain encoded by a nucleotide sequence of SEQ ID NO: 96 and a light chain encoded by a nucleotide sequence of SEQ ID NO:99; or is AB23 and it comprises a heavy chain encoded by a nucleotide sequence of SEQ ID N.98 and a light chain encoded by a nucleotide sequence of SEQ ID NO: 101. Preferably, the isolated antibody is herein named as AB4 and comprises the sequences indicated for antibody AB4 in Tables 1, 2, 5, 6, 11, 12, 15 and 16.

Preferably the isolated antibody or antigen binding fragment thereof of the invention is a human antibody.

More preferably, the isolated antibody or antigen binding fragment thereof is an IgG1 or IgG2 or IgG3 or IgG4 antibody, preferably an IgG1 kappa antibody, an IgG1 lambda antibody, an IgG2 kappa antibody, an IgG2 lambda antibody, an IgG3 kappa antibody or an IgG3 lambda antibody, an IgG4 kappa antibody or an IgG4 lambda antibody, preferably said IgG1 or IgG2 or IgG3 or IgG4 is human IgG1 or human IgG2 or human IgG3 or human IgG4. The invention provides an isolated polynucleotide comprising at least one sequence that encodes the antibody or antigen binding fragment thereof as defined above, preferably said polynucleotide is a cDNA. Preferably, such polynucleotide comprises one or more sequences selected from the group consisting of SEQ ID N.90 to SEQ ID NO.101, in particular as disclosed in Tables 17 and 18. In some embodiments, said polynucleotide comprises:
- a sequence of SEQ ID NO.96 coding for an heavy chain and a sequence of SEQ ID NO.99 coding for a light chain; or
- a sequence of SEQ ID NO.97 coding for an heavy chain and a sequence of SEQ ID NO.100 coding for a light chain; or
- a sequence of SEQ ID NO.98 coding for an heavy chain and a sequence of SEQ ID NO.101 coding for a light chain.

The invention provides a vector comprising the polynucleotide as defined above, preferably said vector is selected from the group consisting of a plasmid, a viral vector, a non-episomal mammalian vector, an expression vector, and a recombinant expression vector.

The invention further provides an isolated cell comprising the polynucleotide as defined above or the vector as defined above, preferably the isolated cell is a hybridoma or a Chinese Hamster Ovary (CHO) cell or a Human Embryonic Kidney cells (HEK293).

The invention further provides the antibody or antigen binding fragment thereof or the isolated polynucleotide or the vector or the isolated cells defined above for use as a medicament, preferably for use in the treatment of lung cancer, more preferably non-small-cell lung cancer (NSCLC).

The invention provides also a pharmaceutical composition comprising the isolated antibody or antigen binding fragment thereof or the isolated polynucleotide or the vector or the isolated cell as defined above and at least one pharmaceutically acceptable carrier, preferably for use in the treatment of a cancer, preferably lung cancer, more preferably NSCLC.

The invention provides a method of treatment of lung cancer, in particular NSCLC, comprising administering to a subject in need thereof a pharmaceutical composition comprising the isolated antibody or antigen binding fragment thereof or the isolated polynucleotide or the vector or the isolated cell as defined above and pharmaceutically acceptable carrier or administering to a subject in need thereof the isolated antibody or antigen binding fragment thereof or the isolated polynucleotide or the vector or the isolated cell as defined above.

The present invention also provides a method for producing an antibody or antigen binding fragment thereof, comprising obtaining the cell as defined above and producing the antibody or antigen binding fragment thereof of the invention.

Nucleic acid molecules encoding the antibody molecules, expression vectors, host cells and methods for making the antibody molecules are also provided. Immunoconjugates, multi- or bispecific antibody molecules and pharmaceutical compositions comprising the antibody molecules are also provided.

The antibody disclosed herein can be used, alone or in combination with other agents or therapeutic modalities, to treat, prevent and/or diagnose lung cancer, in particular NSCLC. Additionally, disclosed herein are methods and compositions comprising a combination of the antibody of the invention with an agent that treats lung cancer, in particular NSCLC and/or an immunotherapeutic agent.

The additional therapeutic agent may be selected from an agent that treat lung cancer, in particular NSCLC, including but not limited to: chemotherapy like cisplatin, carboplatin, paclitaxel, gemcitabine, etoposide, pemetrexed; immunotherapy like Nivolumab (Opdivo), pembrolizumab (Keytruda), and cemiplimab (Libtayo), Atezolizumab (Tecentriq) and durvalumab (Imfinzi), Ipilimumab (Yervoy) and tremelimumab (Imjudo); radiotherapy; targeted therapy like Erlotinib (Tarceva), Afatinib (Gilotrif), Gefitinib (Iressa), Osimertinib (Tagrisso), Dacomitinib (Vizimpro), Sotorasib (Lumakras), Adagrasib (Krazati), Bevacizumab (Avastin), Ramucirumab (Cyramza), Crizotinib (Xalkori), Alectinib (Alecensa), Lorlatinib (Lorbrena), Entrectinib (Rozlytrek), Dabrafenib, Trametinib, Selpercatinib (Retevmo), Tepotinib (Tepmetko), Trastuzumab deruxtecan.

It is also an object of the invention a chimeric antigen receptor (CAR) comprising an antigen-binding fragment of an antibody according to the invention linked to an intracellular domain preferably comprising one or more signaling domains. Preferably, the invention relates to a chimeric antigen receptor (CAR) comprising the scFv of the antibody of the invention linked to an intracellular region comprising the CD3ζ chain, the signaling region of the T cell receptor, and to the two co-stimulatory domains CD28 and 4-1BB. The CAR according to the invention is a relevant tool for targeting malignant cells recognized and bound by the antibody of the invention, for example when expressed in T- cells or NK cells.

A further object of the invention is an antibody-drug conjugates or ADC comprising the antibody or the antigen binding fragment of the invention conjugated with a cytotoxic drug thus advantageously combining the ability of the antibody of the invention to target lung cancer cells, in particular NSCLC cells, with the cancer-killing capabilities of cytotoxic drugs. A further object of the invention is the antibody of the invention for use for inducing antibody-dependent cellular cytotoxicity (ADCC) and/or complement-dependent cytotoxicity (CDC) in a subject affected by a cancer, preferably lung cancer, in particular NSCLC. In particular, in a subject affected by a cancer wherein cancer cells are recognized and bound by the antibodies of the invention. The antibody of the invention can be used alone or in combination with at least one advanced therapy medicinal product (ATMP), such as a tumor-infiltrating lymphocytes (TIL), engineered T Cell receptor (TCR), Chimeric Antigen Receptor (CAR) T or Natural Killer (NK) cell.

A further object of the invention is the antibody of the invention or an antigen binding fragment thereof for use in adoptive cell therapy, in particular Tumor-Infiltrating Lymphocytes (TILs) therapy, engineered T Cell receptors (TCRs) therapy, Chimeric Antigen Receptor (CAR) T Cells therapy or Natural Killer (NK) cells therapy.

### DESCRIPTION OF THE FIGURES

**Figure 1****.** Flow cytometry analysis of 42 antibodies retrieved from MiXCR analysis and produced from HEK293T cells. Binding capacity is represented as a fold increase of Median Fluorescence Intensity (MFI) with respect to the signal of an isotype control antibody.
**Figure 2****.** Percentage of positive live cells labeled with best candidate antibodies AB3, AB4 and AB23. "Isotype" means the percentage of positive cells obtained using an isotype control antibody.
**Figure 3****.** Evaluation of binding capacities on formaldehyde (FA)-fix ed NSCLC cell lines. Binding capacity is represented as a fold increase of Median Fluorescence Intensity (MFI) with respect to the signal of an isotype control antibody.

### DETAILED DESCRIPTION OF THE INVENTION

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

In the present invention "at least 80 % identity" means that the identity may be at least 80%, or 85 % or 90% or 95% or 100% sequence identity to referred sequences. This applies to all the mentioned % of identity. In the present invention "at least 95 % identity" means that the identity may be at least 95%, 96%, 97%, 98%, 99% or 100% sequence identity to referred sequences. This applies to all the mentioned % of identity. In the present invention "at least 98 % identity" means that the identity may be at least 98%, 99% or 100% sequence identity to referred sequences. This applies to all the mentioned % of identity. Preferably, the % of identity relates to the full length of the referred sequence.

The term "antibody" herein is used in the broadest sense understood in the art, including all polypeptides described as. For example, the term "antibody", as used herein, encompasses monoclonal antibodies, polyclonal antibodies, monospecific and multispecific antibodies (e.g., bispecific antibodies), and antibody fragments, so long as the fragment exhibits the desired antigen-binding activity (antigen-binding fragments). The term has its broadest art-recognized meaning and includes all known formats, including, without limitation: bivalent monospecific monoclonal antibodies, bivalent bispecific antibodies, trivalent trispecific antibodies, F(ab) fragments, F(ab)'2 fragments, scFv fragments, diabodies, single domain antibodies, including camelid VHH single domain antibodies, tandabs, and flexibodies. The terms "antigen-binding fragment" of an antibody or equivalently "antigen-binding portion" of an antibody and the like, as used herein, include any naturally occurring, enzymatically obtainable, synthetic, or genetically engineered polypeptide or glycoprotein that comprises a portion of an antibody and that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

As with full antibody molecules, antigen-binding fragments may be monospecific or multispecific (e.g., bispecific). A multispecific antigen-binding fragment of an antibody will typically comprise at least two different variable domains, wherein each variable domain is capable of specifically binding to a separate antigen or to a different epitope on the same antigen.

In particular embodiments, an antigen-binding fragment of an antibody comprises at least one variable domain covalently linked to at least one constant domain. Non-limiting, exemplary configurations of variable and constant domains that may be found within an antigen-binding fragment of an antibody include: (i) VH- CH1; (ii) VH-CH2; (iii) VH-CH3; (iv) VH-CH1-CH2; (v) VH-CH1-CH2-CH3; (vi) VH- CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may in various embodiments consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody may in various embodiments comprise a homo-dimer or hetero-dimer (or other multimer) of any of the variable and constant domain configurations listed above in non-covalent association with one another and/or with one or more monomeric VH or VL domain (e.g., by disulfide bond(s)).

The term "antigen-binding fragment" of an antibody further includes single domain antibodies.

A single-domain antibody is an antibody fragment consisting of a single monomeric variable antibody domain. In some embodiments, the single-domain antibody is derived from the variable domain of the antibody heavy chain from camelids (also termed nanobodies, or VHH fragments). In some embodiments, the single-domain antibody is an autonomous human heavy chain variable domain (aVH) or VNAR fragments derived from sharks. Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain- deleted antibodies, chimeric antibodies, CDR-grafted antibodies, diabodies, triabodies, tetrabodies, minibodies, nanobodies (e.g., monovalent nanobodies, and bivalent nanobodies), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL or VL-VL dimers. Alternatively, the antigen-binding fragment of an antibody may contain a monomeric VH or VL domain.

The antibody or binding molecule of the invention can further be linked to an active substance, preferably a nanoparticle or a radionuclide.

As used herein, the term "antigen binding molecule" refers in its broadest sense to a molecule that specifically binds an antigenic determinant. Examples of antigen binding molecules are antibodies, including antigen-binding antibody fragments, and scaffold antigen binding proteins.

The term "antigen binding moiety" refers to the portion of an antigen binding molecule that specifically binds to an antigenic determinant. Antigen binding moieties include antibodies and antigen-binding fragments thereof, such as scFv, that are capable of specific binding to an antigen on a target cell. In a particular aspect, the antigen binding moiety is able to direct the entity to which it is attached, such as a cell, to a target site.

In addition, antigen binding moieties capable of specific binding to a target cell antigen include scaffold antigen binding proteins as defined herein below, e.g. binding domains which are based on designed repeat proteins or designed repeat domains such as designed ankyrin repeat proteins (DARPins) (see e.g. WO 2002/020565) or Lipocalins (Anticalin). Designed Ankyrin Repeat Proteins (DARPins), which are derived from Ankyrin, which is a family of proteins that mediate attachment of integral membrane proteins to the cytoskeleton. A single ankyrin repeat is a 33-residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028.

In certain embodiments, antibodies and antigen binding molecules provided herein are altered to increase or decrease the extent to which the antigen binding moiety is glycosylated. Glycosylation variants of the molecules may be conveniently obtained by altering the amino acid sequence such that one or more glycosylation sites is created or removed. Where the antigen binding molecule comprises an Fc region, the carbohydrate attached thereto may be altered. In one aspect, variants of antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of antigen binding molecules of the invention include those with bisected oligosaccharides, e.g., in which a biantennary oligosaccharide attached to the Fc region is bisected by GlcNAc. Such variants may have reduced fucosylation and/or improved ADCC function, see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

In certain embodiments, it may be desirable to create cysteine engineered variants of the antibody or antigen binding molecule of the invention, e.g., "thioMAbs," in which one or more residues of the molecule are substituted with cysteine residues. In particular embodiments, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the antibody or antigen binding molecules provided herein may be further modified to contain additional non-proteinaceous moieties that are known in the art and readily available. The moieties suitable for derivatization of the antibody or antigen binding molecule include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-l,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

In another aspect, immunoconjugates of the antibody or antigen binding molecules provided herein may be obtained. An "immunoconjugate" is an antibody conjugated to one or more heterologous molecule(s), including but not limited to a cytotoxic agent.

Preferably, said immunoconjugate is an antibody-drug conjugate (ADC) and it comprises the antibody or fragment thereof conjugated to a cytotoxic drug. Cytotoxic drug is preferably a drug effective against lung cancer, for example a drug approved for the treatment of lung cancer, for example: chemotherapy like cisplatin, carboplatin, paclitaxel, gemcitabine, etoposide, pemetrexed; immunotherapy like Nivolumab (Opdivo), pembrolizumab (Keytruda), and cemiplimab (Libtayo), Atezolizumab (Tecentriq) and durvalumab (Imfinzi), Ipilimumab (Yervoy) and tremelimumab (Imjudo); radiotherapy; targeted therapy like Erlotinib (Tarceva), Afatinib (Gilotrif), Gefitinib (Iressa), Osimertinib (Tagrisso), Dacomitinib (Vizimpro), Sotorasib (Lumakras), Adagrasib (Krazati), Bevacizumab (Avastin), Ramucirumab (Cyramza), Crizotinib (Xalkori), Alectinib (Alecensa), Lorlatinib (Lorbrena), Entrectinib (Rozlytrek), Dabrafenib, Trametinib, Selpercatinib (Retevmo), Tepotinib (Tepmetko), Trastuzumab deruxtecan.

The constant region of an antibody is important in the ability of an antibody to fix the complement and to mediate cell-dependent cytotoxicity. Thus, the isotype of an antibody may be selected on the basis of whether it is desirable for the antibody to mediate cytotoxicity. In certain embodiments, the constant region is an IgG1, IgG2, IgG3, IgG4 constant region.

The invention encompasses antibodies in various embodiments having one or more mutations in the hinge, CH2 or CH3 region which may be desirable, for example, in production, to improve the yield of the desired antibody form. In some embodiments, for example, the antibodies described herein comprise a human IgG4 constant region. In particular embodiments, the IgG4 constant region has a single amino acid substitution in the hinge region of the human IgG4 hinge which reduced Fab arm exchange (Angal et al. (1993) Molecular Immunology 30:105) to levels typically observed using a human IgG1 hinge.

In certain embodiments, the antibody comprises one or more mutations in the constant region that increase serum half-life, including those described in US Patent Nos. 7,083,784, 8,323,962 and Dall'Aqua et al., J. Biol. Chem. 281(33):23514-23524 (2006); Hinton et al., J. Immunology 176:346-356 (2006); Yeung et al., J. Immunology 182:7663-7671 (2009); and Petkova et al., Intn'l Immunology,18: 1759-1769 (2006), incorporated herein by reference in their entireties.

The term "human antibody", as used herein, is intended to include antibodies having variable and constant regions derived from human germline immunoglobulin sequences. The human antibodies featured in the invention may in various embodiments nonetheless include amino acid residues not encoded by human germline immunoglobulin sequences (e.g., mutations introduced by random or site-specific mutagenesis in vitro or by somatic mutation in vivo), for example in the CDRs, and in some embodiments, CDR3. However, the term "human antibody", as used herein, is not intended to include antibodies in which CDR sequences are derived from the germline of another mammalian species, such as a mouse, which have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res.20:6287-6295, incorporated herein by reference in its entirety,) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody." In various embodiments, the isolated antibody also includes an antibody in situ within a recombinant cell. In other embodiments, isolated antibodies are antibodies that have been subjected to at least one purification or isolation step. In various embodiments, an isolated antibody may be substantially free of other cellular material and/or chemicals.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

The present invention includes in various embodiments antibodies and methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations").

Numerous antibodies and antigen-binding fragments may be constructed which comprise one or more individual germline mutations or combinations thereof. In certain embodiments, all of the framework and/or CDR residues within the VH and/or VL domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (i.e., a germline sequence that is different from the germline sequence from which the antibody was originally derived). Furthermore, the antibodies may contain any combination of two or more germline mutations within the framework and/or CDR regions, e.g., wherein certain individual residues are mutated to the corresponding residue of a certain germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The present invention also includes antibodies comprising variants of any of the HCVR, LCVR, and/or CDR amino acid sequences disclosed herein having one or more conservative substitutions. For example, the present invention includes antibodies having HCVR, LCVR, and/or CDR amino acid sequences with, e.g., 10 or fewer, 8 or fewer, 6 or fewer, 4 or fewer, conservative amino acid substitutions relative to any of the HCVR, LCVR, and/or CDR amino acid sequences disclosed herein.

The term "bioequivalent" as used herein, refers to a molecule having similar bioavailability (rate and extent of availability) after administration at the same molar dose and under similar conditions (e.g., same route of administration), such that the effect, with respect to both efficacy and safety, can be expected to be essentially same as the comparator molecule. Two pharmaceutical compositions comprising an antibody are bioequivalent if they are pharmaceutically equivalent, meaning they contain the same amount of active ingredient, in the same dosage form, for the same route of administration and meeting the same or comparable standards. Bioequivalence can be determined, for example, by an in vivo study comparing a pharmacokinetic parameter for the two compositions. Parameters commonly used in bioequivalence studies include peak plasma concentration (Cmax) and area under the plasma drug concentration time curve (AUC).

The invention in certain embodiments relates to antibodies which comprises a heavy chain variable region comprising a sequence selected from the group consisting of: SEQ ID NO: 90, SEQ ID NO: 91 and SEQ ID NO: 92 and a light chain variable region comprising a sequence selected from the group consisting of: SEQ ID NO: 93, SEQ ID NO: 94 and SEQ ID NO:95.

In one embodiment, one or more of the CDRs (or collectively all of the CDRs) have one, two, three, four, five, six or more changes, e.g., amino acid substitutions or deletions, relative to the amino acid sequence shown in Tables 1-14, in particular in Tables 1-2.

In certain embodiments, the antibody includes a substitution in a light chain CDR, e.g., one or more substitutions in a CDR1, CDR2 and/or CDR3 of the light chain.

In certain embodiments, the antibody includes a substitution in a heavy chain CDR, e.g., one or more substitutions in a CDR1, CDR2 and/or CDR3 of the heavy chain.

Preferably, the term "CDR" is a CDR as defined by Kabat, based on sequence comparisons. CDRH1, CDRH2 and CDRH3 denote the heavy chain CDRs, and CDRL1, CDRL2 and CDRL3 denote the light chain CDRs.

In an embodiment, the antibody molecule includes at least one, two, or three CDRs according to Kabat et al., e.g., at least one, two, or three CDRs according to the Kabat definition as set out in Tables 1, 3, 5 and 7, from a heavy chain variable region of an antibody described herein, or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 1, 3-8.

In another embodiment, the antibody molecule includes at least one, two, or three CDRs according to Kabat et al., e.g., at least one, two, or three CDRs according to the Kabat definition as set out in Tables 2, 4, 6 and 8 from a light chain variable region of an antibody described herein, or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 2-8.

In yet another embodiment, the antibody molecule includes all six CDRs according to Kabat et al., e.g., all six CDRs according to the Kabat definition as set out in Tables 1-8, from the heavy and light chain variable regions of an antibody described herein, e.g., an antibody chosen from any of any of AB4, AB3, AB23 as defined in Tables 1-16 or encoded by the nucleotide sequence in Tables 17-18; or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to all six CDRs according to Kabat definition. In one embodiment, the antibody molecule may include any CDR described herein.

In another embodiment, the antibody includes at least one, two, or three CDRs of a heavy chain variable region according to Chothia, AbM, Contact or IMGT definition as disclosed in Tables 9, 11 or 13; or which have at least one amino acid alteration, but not more than two, three or four alterations (e.g., substitutions, deletions, or insertions, e.g., conservative substitutions).

In another embodiment, the antibody includes at least one, two, or three CDRs of a light chain variable region according to Chothia, AbM, Contact or IMGT definition as disclosed in Table 10, 12 or 14; or which have at least one amino acid alteration, but not more than two, three or four alterations (e.g., substitutions, deletions, or insertions, e.g., conservative substitutions).

In certain embodiments, the antibody molecule includes a combination of CDRs defined according to the Kabat, Chothia, AbM, Contact or IMGT definition.

Preferred antibodies are antibodies AB4, AB3 and AB23 as defined in Tables 1-16 or encoded by the nucleotide sequence in Tables 17 and 18.

In one embodiment, the antibody includes:
(i) a heavy chain variable region (VH) including a VHCDR1 amino acid sequence of SEQ ID NO: 1; a VHCDR2 amino acid sequence of SEQ ID NO: 2; and a VHCDR3 amino acid sequence chosen from any one of SEQ ID NO:3, 4 and 5; and
(ii) a light chain variable region (VL) including a VLCDR1 amino acid sequence chosen from any one of SEQ ID NO: 6, 9 and 12, a VLCDR2 amino acid sequence chosen from any one of SEQ ID NO: 7, 10 and 13, and a VLCDR3 amino acid sequence chosen from SEQ ID NO: 8, 11 and 14.

In one embodiment, the light or the heavy chain variable framework (e.g., the region encompassing at least FR1, FR2, FR3, and optionally FR4) of the antibody molecule can be chosen from: (a) a light or heavy chain variable framework including at least 80%, 85%, 87% 90%, 92%, 93%, 95%, 97%, 98%, or preferably 100% of the amino acid residues from a human light or heavy chain variable framework, e.g., a light or heavy chain variable framework residue from a human mature antibody, a human germline sequence, or a human consensus sequence; (b) a light or heavy chain variable framework including from 20% to 80%, 40% to 60%, 60% to 90%, or 70% to 95% of the amino acid residues from a human light or heavy chain variable framework, e.g., a light or heavy chain variable framework residue from a human mature antibody, a human germline sequence, or a human consensus sequence; (c) a non-human framework (e.g., a rodent framework); or (d) a non-human framework that has been modified, e.g., to remove antigenic or cytotoxic determinants, e.g., deimmunized, or partially humanized. In one embodiment, the light or heavy chain variable framework region (particularly FR1, FR2 and/or FR3) includes a light or heavy chain variable framework sequence at least 70, 75, 80, 85, 87, 88, 90, 92, 94, 95, 96, 97, 98, 99% identical or identical to the frameworks of a VL or VH segment of a human germline gene. Preferably, the light and the heavy chain variable framework are as described in Tables 3, 5 and 7 for the heavy chain and 4, 6 or 8 for the light chain.

In certain embodiments, the antibody molecule comprises a heavy chain variable domain having at least one, two, three, four, five, six, seven, ten, fifteen, twenty or more changes, e.g., amino acid substitutions or deletions with respect to the sequence of: SEQ ID NO: 90, SEQ ID NO: 91 or SEQ ID NO: 92.

In certain embodiments, the antibody molecule comprises a light chain variable domain having at least one, two, three, four, five, six, seven, ten, fifteen, twenty or more changes, e.g., amino acid substitutions or deletions with respect to the sequence of SEQ ID NO: 93, SEQ ID NO: 94 or SEQ ID NO:95.

In one embodiment, the heavy or light chain variable region, or both, of the antibody molecule includes an amino acid sequence encoded by a nucleic acid sequence described herein or a nucleic acid that hybridizes to a nucleic acid sequence described herein, in particular a nucleic acid sequence as shown in Tables 17 and 18 or its complement, e.g., under low stringency, medium stringency, or high stringency, or other hybridization condition described herein.

In another embodiment, the antibody molecule comprises at least one, two, three, or four antigen-binding regions, e.g., variable regions, having an amino acid sequence as set forth in anyone of Tables 1-16 or a sequence substantially identical thereto (e.g., a sequence at least about 85%, 90%, 95%, 99% or more identical thereto, or which differs by no more than 1, 2, 5, 10, or 15 amino acid residues from the sequences shown in Tables 1-16).

In another embodiment, the antibody molecule includes a VH and/or VL domain encoded by a nucleic acid having a nucleotide sequence as set forth in Tables 17 and 18or a sequence substantially identical thereto (e.g., a sequence at least about 85%, 90%, 95%, 99% or more identical thereto, or which differs by no more than 3, 6, 15, 30, or 45 nucleotides from the sequences shown in Tables 17-18).

In yet other embodiments, the antibody molecule has a heavy chain constant region (Fc) chosen from, e.g., the heavy chain constant regions of IgG1, IgG2, IgG3, IgG4, IgM, IgA1, IgA2, IgD, and IgE; particularly, chosen from, e.g., the heavy chain constant regions of IgG1, IgG2, IgG3, and IgG4, more particularly, the heavy chain constant region of IgG1 or IgG4 (e.g., human IgG1, IgG2 or IgG4). In one embodiment, the heavy chain constant region is human IgG1. In another embodiment, the antibody molecule has a light chain constant region chosen from, e.g., the light chain constant regions of kappa or lambda. In one embodiment, the constant region is altered, e.g., mutated, to modify the properties of the antibody (e.g., to increase or decrease one or more of: Fc receptor binding, antibody glycosylation, the number of cysteine residues, effector cell function, complement function, half-life, aggregation and stability). In certain embodiments, the antibody comprises a human IgG4 mutated.

In one embodiment, antibody is isolated or recombinant.

In one embodiment, the antibody is a humanized or human antibody molecule.

The antibody of the invention may have framework sequences from any species. Preferably, it may have a mouse or human framework As used herein the term "framework (FR) amino acid residues" refers to those amino acids in the framework region of an immunoglobulin chain. The term "framework region" or "FR region" as used herein, includes the amino acid residues that are part of the variable region, but are not part of the CDRs (e.g., using the Kabat definition of CDRs).

A monoclonal antibody comprising the CDRs sequences of any of the antibody herein described is also within the scope of the invention.

Methods for producing a monoclonal antibody with the CDR sequences as mentioned above are known in the art and include the introduction of the nucleic acid sequences encoding the CDRs into suitable expression vectors encoding the desired framework sequences.

The antibody according to the invention may form a homo- or heterodimer or a homo- or heteromultimer, whereby "dimer" and "multimer" means that two and at least three antibodies, respectively, may combine to form a complex. The prefix "homo" means that a complex may be formed of identical antibody molecules, whereby the prefix "hetero" means that a complex may be formed of different antibody molecules. In general, the term "antibody" is intended to comprise all above-mentioned immunoglobulin isotypes, i.e. the antibody may be an IgA, IgD, IgE, IgG or IgM antibody, including any subclass of these isotypes. Preferably, the antibody is an IgG antibody, more preferred the antibody is an IgGI antibody. Since the antibody may be expressed and produced recombinantly, the antibody may also comprise two different constant regions of heavy chains, e.g. one IgGI and one IgG2 heavy chain, or heavy chains from different species. However, the heavy chains preferably are from the same species.

To generate whole antibodies, PCR primers including VH or VL nucleotide sequences, a restriction site, and a flanking sequence to protect the restriction site can be used to amplify the VH or VL sequences in scFv clones or other clones. Utilizing cloning techniques known to those of skill in the art, the PCR amplified VH domains can be cloned into vectors expressing a heavy chain constant region, e.g., the human gamma 4 constant region, and the PCR amplified VL domains can be cloned into vectors expressing a light chain constant region, e.g., human kappa or lambda constant regions. In certain embodiments, the vectors for expressing the VH or VL domains comprise a promoter, a secretion signal, a cloning site for the variable region, constant domains, and a selection marker such as neomycin. The VH and VL domains can also be cloned into one vector expressing the necessary constant regions. The heavy chain conversion vectors and light chain conversion vectors are then co-transfected into cell lines to generate stable or transient cell lines that express full-length antibodies, e.g., IgG, using techniques known to those of skill in the art.

The invention also features a nucleic acid molecule that comprises one or both nucleotide sequences that encode heavy and light chain variable regions, CDRs, framework regions of the antibody, as described herein. In certain embodiments, the nucleotide sequence that encodes the antibody molecule is codon optimized. For example, the invention features a first and second nucleic acid encoding heavy and light chain variable regions, respectively, of an antibody molecule chosen from one or more of, e.g., any of AB4, AB3, AB23 as defined in Tables 1-16 or encoded by the nucleotide sequence in Tables 17 and 18, or a sequence substantially identical thereto. For example, the nucleic acid can comprise a nucleotide sequence as set forth in Tables 17 and 18, or a sequence substantially identical thereto (e.g., a sequence at least about 85%, 90%, 95%, 99% or more identical thereto, or which differs by no more than 3, 6, 15, 30, or 45 nucleotides from the sequences shown in Tables 17 and 18).

In other embodiments, the nucleic acid molecule comprises a nucleotide sequence that encodes a heavy chain variable domain and/or a heavy chain constant region comprising the amino acid sequence of the heavy chain of any of antibody AB4, AB3, AB23 as defined in Table 15 or a nucleotide sequence as defined in Table 17; or a sequence substantially identical (e.g., a sequence at least about 85%, 90%, 95%, 99% or more identical) to any of the aforesaid sequences.

In other embodiments, the nucleic acid molecule comprises a nucleotide sequence that encodes a light chain variable domain and/or a light chain constant region comprising the amino acid sequence of the light chain any of AB4, AB3, AB23 as defined in Table 16 or a nucleotide sequence as defined in Table 18, or a sequence substantially identical (e.g., a sequence at least about 85%, 90%, 95%, 99% or more identical) to any of the aforesaid sequences.

The aforesaid nucleotide sequences encoding the herein disclosed heavy and light chain variable domain and constant regions can be present in a separate nucleic acid molecule, or in the same nucleic acid molecule. In certain embodiments, the nucleic acid molecules comprise a nucleotide sequence encoding a leader sequence.

In certain embodiments, the nucleic acid molecule comprises a nucleotide sequence encoding at least one, two, or three CDRs from a heavy chain variable region having an amino acid sequence as set forth in Tables 1, 3, 5, 7, 9, 11 or 13, or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule comprises a nucleotide sequence encoding at least one, two, or three CDRs, or hypervariable loops, from a light chain variable region having an amino acid sequence as set forth in Tables 2, 4, 6, 8, 10, 12 or 14 or a sequence substantially homologous thereto (e.g., a sequence at least about 85%, 90%, 95%, 99% or more identical thereto, and/or having one, two, three or more substitutions, insertions or deletions, e.g., conserved substitutions).

In yet another embodiment, the nucleic acid molecule comprises a nucleotide sequence encoding at least one, two, three, four, five, or six CDRs from heavy and light chain variable regions having an amino acid sequence as set forth in Tables 15 and 16 or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule includes one or more heavy chain framework region (e.g., any of VHFW1 (type a), VHFW1 (type b), VHFW1 (type c), VHFW1 (type d), VHFW2 (type a), VHFW2 (type a'), VHFW2 (type b), VHFW2 (type c), VHFW2 (type d), VHFW2 (type e), VHFW3 (type a), VHFW3 (type b), VHFW3 (type c), VHFW3 (type d), VHFW3 (type e), or VHFW4, or any combination thereof, e.g., a framework combination as described herein for any of AB4, AB3, AB23 as defined in Tables 3-14, or a sequence substantially identical thereto. For example, the nucleic acid molecule can comprise a nucleotide sequence as set forth in Table 17, or a sequence substantially identical thereto (e.g., a sequence at least about 85%, 90%, 95%, 99% or more identical thereto, or which differs by no more than 3, 6, 15, 30, or 45 nucleotides from the sequences shown in Table 17).

In another embodiment, the nucleic acid molecule includes one or more light chain framework region (e.g., any of VLFW1 (type a), VLFW1 (type b), VLFW1 (type c), VLFW1 (type d), VLFW1 (type e), VLFW1 (type f), VLFW2 (type a), VLFW2 (type c), VLFW3 (type a), VLFW3 (type b), VLFW3 (type c), VLFW3 (type d), VLFW3 (type e), VLFW3 (type f), VLFW3 (type g), or VLFW4, or any combination thereof, e.g., a framework combination as described herein for of any of AB4, AB3, AB23 as defined in Tables 3-14, or a sequence substantially identical thereto. For example, the nucleic acid molecule can comprise a nucleotide sequence as set forth in Table 18, or a sequence substantially identical thereto (e.g., a sequence at least about 85%, 90%, 95%, 99% or more identical thereto, or which differs by no more than 3, 6, 15, 30, or 45 nucleotides from the sequences shown in Table 17).

In another embodiment, the nucleic acid molecule includes one or more heavy chain framework region and one or more light chain framework region as described herein. The heavy and light chain framework regions may be present in the same vector or separate vectors.

In another aspect, the application features host cells and vectors containing the nucleic acids described herein or modified for codon optimization according to known methods. The nucleic acids may be present in a single vector or separate vectors present in the same host cell or separate host cell. The host cell can be a eukaryotic cell, e.g., a mammalian cell, an insect cell, a yeast cell, or a prokaryotic cell, e.g., E. coli. For example, the mammalian cell can be a cultured cell or a cell line. Exemplary mammalian cells include lymphocytic cell lines (e.g., NSO), Chinese hamster ovary cells (CHO), COS cells, oocyte cells, and cells from a transgenic animal, e.g., mammary epithelial cell.

Generally, expression vectors are plasmids which are used to introduce a desired nucleic acid sequence, such as a gene, into a target cell, resulting in the transcription and translation of the protein encoded by the nucleic acid sequence, i.e. the chimeric antigen receptor, the antibody or the binding molecule. Therefore, the expression vector in general comprises regulatory sequences, such as promoter and enhancer regions, as well as a polyadenylation site in order to direct efficient transcription of the nucleic acid sequence on the expression vector. The expression vector may further comprise additional necessary or useful regions, such as a selectable marker for selection in eukaryotic or prokaryotic cells, a purification tag for the purification of the resulting protein, a multiple cloning site or an origin of replication.

Usually, the expression vector may be a viral or a non- viral vector. In general, various kinds of viral vectors, such as retroviral vectors, e.g. lentiviral or adenoviral vectors, or plasmids may be used. In a preferred embodiment, the expression vector according to aspect five is a viral vector. In a more preferred embodiment, the expression vector is a lentiviral vector. In another aspect, the invention provides, compositions, e.g., pharmaceutical compositions, which include a pharmaceutically acceptable carrier, excipient or stabilizer, and at least one of the antibody molecules described herein. In one embodiment, the composition, e.g., the pharmaceutical composition, includes a combination of the antibody molecule and one or more agents, e.g., a therapeutic agent or other antibody molecule, as described herein. In one embodiment, the antibody molecule is conjugated to a label or a therapeutic agent. The disclosure provides pharmaceutical compositions comprising such antibody, and methods of using these compositions.

The antibody is administered to the subject in various embodiments in a formulation comprising suitable carriers, excipients, and other agents to provide improved transfer, delivery, tolerance, and the like, and suitable for an intravenous or subcutaneous injection. The injectable preparations may be prepared by methods publicly known. For example, injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 20 or 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injectable preparation thus prepared can be filled in an appropriate ampoule.

The content of the antibody or antigen binding fragment thereof in the pharmaceutical composition is not limited as far as it is useful for treatment or prevention, but preferably contains 0.0000001-10% by weight per total composition. Further, the antibody or antigen binding fragment thereof described herein are preferably employed in a carrier. The choice of carrier may depend upon route of administration and concentration of the active agent(s) and the carrier may be in the form of a lyophilised composition or an aqueous solution.

Generally, an appropriate amount of a pharmaceutically acceptable salt is used in the carrier to render the composition isotonic. Examples of the carrier include but are not limited to saline, Ringer's solution and dextrose solution. Preferably, acceptable excipients, carriers, or stabilisers are non-toxic at the dosages and concentrations employed, including buffers such as citrate, phosphate, and other organic acids; salt-forming counter- ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). Suitable carriers and their formulations are described in greater detail in Remington's Pharmaceutical Sciences, 17th ed., 1985, Mack Publishing Co. The composition may also contain at least one further active compound, such as a chemotherapeutic agent.

Preferably, the antibody or antigen binding fragment thereof is included in an effective amount. The term "effective amount" refers to an amount sufficient to induce a detectable therapeutic response in the subject to which the pharmaceutical composition is to be administered.

The antibody according to the invention can be administered to the subject using any acceptable device or mechanism. For example, the administration can be accomplished using a syringe and needle or with a reusable pen and/or autoinjector delivery device. According to the invention, "subject" means a human subject or human patient.

In an embodiment, the present antibodies are for use for the treatment of a cancer. Said cancer can be selected from lung cancer, skin cancer, colon cancer, prostate cancer, pancreas cancer, breast cancer, brain cancer, stomach cancer and liver cancer. In particular, it is a cancer wherein cancer cells are bound by the antibodies of the invention. Preferably, it is a cancer wherein cancer cells express an antigen present also in lung cancer cells.

In a preferred embodiment, the present antibodies are for use for the treatment of lung cancer, in particular NSCLC.

Accordingly, in another aspect, a method of treating lung cancer, in particular NSCLC, in a subject is provided. The method comprises administering to the subject an antibody molecule disclosed herein (e.g., a therapeutically effective amount of an antibody molecule), alone or in combination with one or more agents or procedures, such that the cancer in the subject is treated.

In one embodiment, the antibody molecule inhibits, reduce or neutralize or block lung cancer in the subject. The subject can be a mammal, e.g., a primate, preferably a higher primate, e.g., a human (e.g., a patient having, or at risk of having, a disorder described herein). In one embodiment, the subject is in need of inhibiting, reducing, neutralizing or blocking lung cancer. In one embodiment, the subject has, or is at risk of, having lung cancer, in particular NSCLC.

For "lung cancer" it is herein intended a type of cancer that begins in one or both lungs. For "NSCLC cancer" or "non-small cell lung cancer" it is herein intended any type of epithelial lung cancer other than small-cell lung cancer. Exemplary types of NSCLC cancers are squamous-cell carcinoma, large-cell carcinoma, and adenocarcinoma.

The term "Antibody-dependent cellular cytotoxicity (ADCC)", as used herein, is the killing of a cell bound and marked by antibodies by a cytotoxic effector cell, such as natural killer (NK) cells.

The term "Complement-dependent cytotoxicity (CDC)" as used herein is the triggering of the classical complement pathway by the binding of antibodies to surface antigen on target cells, resulting in the killing of the target cell.

The term "Chimeric antigen receptor" (CAR), as used herein, refers to synthetic receptors comprising a targeting moiety that is associated with one or more signaling domains in a single fusion molecule. In general, the binding moiety of a CAR comprises scFv, but it may also comprise other binding entities. Binding moieties based on receptor or ligand domains have also been used successfully. The signaling domains for CARs can be derived from the cytoplasmic region of the CD3ζ or the Fc receptor gamma chains, but may also be derived from other cytoplasmic regions. First generation CARs have been shown to successfully redirect T-cell cytotoxicity. Signaling domains from co-stimulatory molecules, as well as transmembrane and hinge domains have been added to form CARs of second and third generations, leading to some successful therapeutic trials in humans, where T-cells could be redirected against malignant cells expressing CD19 (Porter DL et al., N Eng J Med, 2011).

The term "adoptive cell therapy" as used herein refers to a treatment that uses the cells of the human immune system to eliminate cancer. In particular, cells of the immune system, such as T cells or natural killer cells, may be genetically engineered to enhance their cancer-fighting capabilities. Examples of adoptive cell therapies are: Tumor-Infiltrating Lymphocyte (TIL) Therapy, Engineered T Cell Receptor (TCR) Therapy, Chimeric Antigen Receptor (CAR) T Cell Therapy and Natural Killer (NK) Cell Therapy. The antibody of the invention or an antigen binding fragment thereof can be advantageously used in any of these kinds of adoptive cell therapy, in particular by engineering cells of the immune system, such as T cells and NK cells, with the antibody of the invention or an antigen binding thereof thus providing them with the ability to recognize lung cancer cells, in particular NSCLC cancer cells.

Antibodies described herein can be used alone or in combination in the methods of the invention.

### SEQUENCES

The sequences of the CDRs of the 3 antibodies of the invention are reported in Tables 1-2 below.

| **Table 1: VH CDR Sequences of exemplified antibodies** | | | |
|---|---|---|---|
| **Antibody** | **CDR1** | **CDR2** | **CDR3** |
| **AB3** | SSNWWS (SEQ ID NO. 1) | EIYHSGSTNYNPSLKS (SEQ ID NO. 2) | DKMVRGLIDYYYYGLD V(SEQ ID NO. 3 ) |
| **AB4** | SSNWWS (SEQ ID NO. 1) | EIYHSGSTNYNPSLKS (SEQ ID NO. 2) | ARLHFDY (SEQ ID NO. 4) |
| **AB23** | SSNWWS (SEQ ID NO. 1) | EIYHSGSTNYNPSLKS (SEQ ID NO. 2) | SYFDSGMDV (SEQ ID NO. 5) |

| **Table 2: VL CDR Sequences of exemplified antibodies** | | | |
|---|---|---|---|
| **Antibody** | **CDR1** | **CDR2** | **CDR3** |
| **AB3** | GASQSVSSSYLA (SEQ ID NO. 6) | DASSRAT (SEQ ID NO. 7) | QQYGSSLT (SEQ ID NO. 8) |
| **AB4** | RSSQSLLHSNGYNYLD (SEQ ID NO. 9) | LGSNRAS (SEQ ID NO. 10) | MQALQTPPRFT (SEQ ID NO. 11) |
| **AB23** | RASQSVSSNLA (SEQ ID NO. 12) | GA | QQYNKWP (SEQ ID NO. 13) |

CDR definition is also provided using annotation tool from http://www.abysis.org/ based on full VH and VL amino acid sequences as defined in Tables 15 and 16.

For example, the VH amino acid sequence of any antibody disclosed herein is plugged into the annotation tool and Kabat defined CDR sequences are provided.

In the following Tables 3-8 Kabat defined CDR and framework regions of the heavy chain variable region (VH) and light chain variable region (VL) are reported for each antibody.

In the Tables, HFR means framework region of heavy chain and LFR means framework region of light chain.

**Table 3. Antibody AB3; VH CDR:**

| **Region** | **Sequence Fragment** | **Residues** | **Length** |
|---|---|---|---|
| **HFR1** | QVQLQESGPGLVKPPGTLSLTCAVSGGSIS (SEQ ID NO. 14) | 01- 30 | 30 |
| **CDR-H1** | SSNWWS (SEQ ID NO.1) | 31 - 36 | 6 |
| **HFR2** | WVRQPPGKGLEWIG (SEQ ID NO. 15) | 37 - 50 | 14 |
| **CDR-H2** | EIYHSGSTNYNPSLKS (SEQ ID NO. 2) | 51 - 66 | 16 |
| **HFR3** | RVTISVDTSKNQISLQLSSVTAADTAVYYCAR (SEQ ID NO. 16) | 67 - 98 | 32 |
| **CDR-H3** | DKMVRGLIDYYYYGLDV (SEQ ID NO. 3) | 99 - 115 | 17 |
| **HFR4** | WGQGTTVTVSS (SEQ ID NO. 17) | 116 - 126 | 11 |
| | | | 126 |

**Table 4. Antibody AB3; VL CDR:**

| **Region** | **Sequence Fragment** | **Residues** | **Length** |
|---|---|---|---|
| **LFR1** | EIVLTQSPATLSLSPGERATLSC (SEQ ID NO. 18) | 01 - 23 | 23 |
| **CDR-L1** | GASQSVSSSYLA (SEQ ID NO. 6) | 24 - 35 | 12 |
| **LFR2** | WYQQKPGLAPRLLIY (SEQ ID NO. 19) | 36 - 50 | 15 |
| **CDR-L2** | DASSRAT (SEQ ID NO. 7) | 51 - 57 | 7 |
| **LFR3** | GIPDRFSGSGSGTDFTLTISRLEPEDFAVYYC (SEQ ID NO. 20) | 58 - 89 | 32 |
| **CDR-L3** | QQYGSSLT (SEQ ID NO. 8) | 90 - 97 | 8 |
| **LFR4** | FGQGTKVEIK (SEQ ID NO. 21) | 98 - 107 | 10 |
| | | | 107 |

**Table 5. Antibody AB4; VH CDR:**

| **Region** | **Sequence Fragment** | **Residues** | **Length** |
|---|---|---|---|
| **HFR1** | QVQLQESGPGLVKPPGTLSLTCAVSGGSIS (SEQ ID NO. 14) | 01 - 30 | 30 |
| **CDR-H1** | SSNWWS (SEQ ID NO. 1) | 31 - 36 | 6 |
| **HFR2** | WVRQPPGKGLEWIG (SEQ ID NO. 15) | 37 - 50 | 14 |
| **CDR-H2** | EIYHSGSTNYNPSLKS (SEQ ID NO. 2) | 51 - 66 | 16 |
| **HFR3** | RVTISVDKSKNHFSLYVSSVTAADTAVYYCAR (SEQ ID NO. 22) | 67 - 98 | 32 |
| **CDR-H3** | ARLHFDY (SEQ ID NO. 4) | 99 - 105 | 7 |
| **HFR4** | WGQGTLVTVSS (SEQ ID NO. 23) | 106 - 116 | 11 |

**Table 6. Antibody AB4; VL CDR:**

| **Region** | **Sequence Fragment** | **Residues** | **Length** |
|---|---|---|---|
| **LFR1** | DIVMTQSPLSLPVTPGEPASISC (SEQ ID NO. 24) | 01 - 23 | 23 |
| **CDR-L1** | RSSQSLLHSNGYNYLD (SEQ ID NO. 9) | 24 - 39 | 16 |
| **LFR2** | WYLQKPGQSPQLLIY (SEQ ID NO. 25) | 40 - 54 | 15 |
| **CDR-L2** | LGSNRAS (SEQ ID NO. 10) | 55 - 61 | 7 |
| **LFR3** | GVPDRFSGSGSGTDFTLKISRVEAEDVGVYYC (SEQ ID NO. 26) | 62 - 93 | 32 |
| **CDR-L3** | MQALQTPPRFT (SEQ ID NO. 11) | 94 - 104 | 11 |
| **LFR4** | FGPGTKVDIK (SEQ ID NO.27) | 105 - 114 | 10 |
| | | | 114 |

**Table 7. Antibody AB23; VH CDR:**

| **Region** | **Sequence Fragment** | **Residues** | **Length** |
|---|---|---|---|
| **HFR1** | QVQLQESGPGLVKPPGTLSLTCAVSGGSIS (SEQ ID NO. 14) | 01 - 30 | 30 |
| **CDR-H1** | SSNWWS (SEQ ID NO. 1) | 31 - 36 | 6 |
| **HFR2** | WVRQPPGKGLEWIG (SEQ ID NO. 15) | 37 - 50 | 14 |
| **CDR-H2** | EIYHSGSTNYNPSLKS (SEQ ID NO. 2) | 51 - 66 | 16 |
| **HFR3** | RVTISVDTSKNHFYLNLSSVTAADTALYYCAR (SEQ ID NO. 28) | 67 - 98 | 32 |
| **CDR-H3** | SYFDSGMDV (SEQ ID NO. 5) | 99 - 107 | 9 |
| **HFR4** | WGQGTTVTVSS (SEQ ID NO. 17) | 108 - 118 | 11 |
| | | | 118 |

**Table 8. Antibody AB23; VL CDR:**

| **Region** | **Sequence Fragment** | **Residues** | **Length** |
|---|---|---|---|
| **LFR1** | EIVMTQSPATLSVSPGERATLSC (SEQ ID NO. 29) | 1-26 | 26 |
| **CDR-L1** | RASQSVSSNLA (SEQ ID NO. 12) | 27 -32 | 6 |
| **LFR2** | WYQQKLGQASRLLIY (SEQ ID NO. 30) | 33 -49 | 17 |
| **CDR-L2** | GA | 50-51 | 3 |
| **LFR3** | SGSGSGTEFTLTISSLQSEDFAVYYC (SEQ ID NO. 31) | 52-77 | 36 |
| **CDR-L3 (V gene only)** | QQYNKWP (SEQ ID NO. 13) | 78-84 | 7 |
| | | | 95 |

In addition, the VH amino acid sequence of any antibody disclosed herein may also be plugged into the annotation tool and the CDR sequences defined according to Chothia, AbM, Kabat, Contact and IMGT definitions are provided.

Table 9 below shows CDR sequences in Chothia, AbM, Kabat, Contact and IMGT for VH of Antibody AB3.

**Table 9. Antibody AB3; VH CDR:**

| **Regio n** | **Definitio n** | **Sequence Fragment** | **Residue s** | **Lengt h** |
|---|---|---|---|---|
| **HFR1** | Chothia | QVQLQESGPGLVKPPGTLSLTCAVS----- (SEQ. NO. 32) | 1 - 25 | 25 |
| | AbM | QVQLQESGPGLVKPPGTLSLTCAVS----- (SEQ. NO. 32) | 1 - 25 | 25 |
| | Kabat | QVQLQESGPGLVKPPGTLSLTCAVSGGSIS (SEQ. NO.14) | 1 - 30 | 30 |
| | Contact | QVQLQESGPGLVKPPGTLSLTCAVSGGSI- (SEQ. NO.33) | 1 - 29 | 29 |
| | IMGT | QVQLQESGPGL VKPPGTLSL TCA VS----- (SEQ. NO.32) | 1 - 25 | 25 |
| **CDR-H1** | Chothia | GGSISSSN --- (SEQ. NO.34) | 26 - 33 | 8 |
| | AbM | GGSISSSNWWS (SEQ. NO.35) | 26 - 36 | 11 |
| | Kabat | -----SSNWWS (SEQ. NO.1) | 31 - 36 | 6 |
| | Contact | ----SSSNWWS (SEQ. NO.36) | 30 - 36 | 7 |
| | IMGT | GGSISSSNW-(SEQ. NO.37) | 26 - 34 | 9 |
| **HFR2** | Chothia | WWSWVRQPPGKGLEWIGEI (SEQ. NO.38) | 34 - 52 | 19 |
| | AbM | ---WVRQPPGKGLEWIG-(SEQ. NO.15) | 37 - 50 | 14 |
| | Kabat | ---WVRQPPGKGLEWIG-- SEQ. NO.15) | 37 - 50 | 14 |
| | Contact | --- WVRQPPGKGLE ----- (SEQ. NO.39) | 37 - 47 | 11 |
| | IMGT | -WSWVRQPPGKGLEWIGE- (SEQ. NO.40) | 35 - 51 | 17 |
| **CDR-H2** | Chothia | -----YHSGS--------- (SEQ. NO.41) | 53 - 57 | 5 |
| | AbM | ---EIYHSGSTN------- (SEQ. NO.42) | 51 - 59 | 9 |
| | Kabat | ---EIYHSGSTNYNPSLKS (SEQ. NO.2) | 51 - 66 | 16 |
| | Contact | WIGEIYHSGSTN------- (SEQ. NO.43) | 48 - 59 | 12 |
| | IMGT | ----IYHSGST-------- (SEQ. NO.44) | 52 - 58 | 7 |
| **HFR3** | Chothia | TNYNPSLKSRVTISVDTSKNQISLQLSSVTAADTAVY YCAR (SEQ. NO.45) | 58 - 98 | 41 |
| | AbM | -- YNPSLKSRVTISVDTSKNQISLQLSSVTAADTAVYYC AR (SEQ. NO.46) | 60 - 98 | 39 |
| | Kabat | ---------RVTISVDTSKNQISLQLSSVTAADTAVYYCAR (SEQ. NO.16) | 67 - 98 | 32 |
| | Contact | -YNPSLKSRVTISVDTSKNQISLQLSSVTAADTAVYYC- - (SEQ. NO.47) | 60 - 96 | 37 |
| | IMGT | - NYNPSLKSRVTISVDTSKNQISLQLSSVTAADTAVYY C-- (SEQ. NO.48) | 59 - 96 | 38 |
| **CDR-H3** | Chothia | --DKMVRGLIDYYYYGLDV (SEQ. NO.3) | 99- 115 | 17 |
| | AbM | --DKMVRGLIDYYYYGLDV (SEQ. NO.3) | 99 - 115 | 17 |
| | Kabat | --DKMVRGLIDYYYYGLDV (SEQ. NO.3) | 99 - 115 | 17 |
| | Contact | ARDKMVRGLIDYYYYGLD- (SEQ. NO.49) | 97 - 114 | 18 |
| | IMGT | ARDKMVRGLIDYYYYGLDV (SEQ. NO.50) | 97 - 115 | 19 |
| **HFR4** | Chothia | -WGQGTTVTVSS (SEQ ID NO. 17) | 116-126 | 11 |
| | AbM | -WGQGTTVTVSS (SEQ ID NO. 17) | 116-126 | 11 |
| | Kabat | -WGQGTTVTVSS (SEQ ID NO. 17) | 116-126 | 11 |
| | Contact | VWGQGTTVTVSS (SEQ ID NO. 51) | 115-126 | 12 |
| | IMGT | -WGQGTTVTVSS (SEQ ID NO. 17) | 116-126 | 11 |

Table 10 below shows CDR sequences in Chothia , AbM, Kabat, Contact and IMGT for VL of Antibody AB3.

**Table 10. Antibody AB3; VL CDR:**

| **Regio n** | **Definitio n** | **Sequence Fragment** | **Residue s** | **Lengt h** |
|---|---|---|---|---|
| **LFR1** | Chothia | EIVLTQSPATLSLSPGERATLSC------ (SEQ ID NO. 18) | 1 - 23 | 23 |
| | AbM | EIVLTQSPATLSLSPGERATLSC------ (SEQ ID NO. 18) | 1 - 23 | 23 |
| | Kabat | EIVLTQSPATLSLSPGERATLSC------ (SEQ ID NO. 18) | 1 - 23 | 23 |
| | Contact | EIVLTQSPATLSLSPGERATLSCGASQSV (SEQ ID NO. 52) | 1 - 29 | 29 |
| | IMGT | EIVLTQSPATLSLSPGERATLSCGAS--- (SEQ ID NO. 53) | 1 - 26 | 26 |
| **CDR-L1** | Chothia | GASQSVSSSYLA-- (SEQ ID NO. 6) | 24 - 35 | 12 |
| | AbM | GASQSVSSSYLA-- (SEQ ID NO. 6) | 24 - 35 | 12 |
| | Kabat | GASQSVSSSYLA-- (SEQ ID NO. 6) | 24 - 35 | 12 |
| | Contact | ------SSSYLAWY (SEQ ID NO. 54) | 30 - 37 | 8 |
| | IMGT | --- QSVSSSY---- (SEQ ID NO. 55) | 27 - 33 | 7 |
| **LFR2** | Chothia | --WYQQKPGLAPRLLIY (SEQ ID NO. 19) | 36 - 50 | 15 |
| | AbM | --WYQQKPGLAPRLLIY (SEQ ID NO. 19) | 36 - 50 | 15 |
| | Kabat | --WYQQKPGLAPRLLIY (SEQ ID NO. 19) | 36 - 50 | 15 |
| | Contact | ----QQKPGLAPR---- (SEQ ID NO. 56) | 38 - 46 | 9 |
| | IMGT | LAWYQQKPGLAPRLLIY (SEQ ID NO. 57) | 34 - 50 | 17 |
| **CDR-L2** | Chothia | ----DASSRAT (SEQ ID NO. 7) | 51 - 57 | 7 |
| | AbM | ----DASSRAT (SEQ ID NO. 7) | 51 - 57 | 7 |
| | Kabat | ----DASSRAT (SEQ ID NO. 7) | 51 -57 | 7 |
| | Contact | LLIYDASSRA- (SEQ ID NO. 58) | 47 - 56 | 10 |
| | IMGT | ----DA----- | 51 - 52 | 2 |
| **LFR3** | Chothia | -----GIPDRFSGSGSGTDFTLTISRLEPEDFA VYYC (SEQ ID NO. 20) | 58 - 89 | 32 |
| | AbM | -----GIPDRFSGSGSGTDFTLTISRLEPEDFA VYYC (SEQ ID NO. 20) | 58 - 89 | 32 |
| | Kabat | -----GIPDRFSGSGSGTDFTLTISRLEPEDFA VYYC (SEQ ID NO. 20) | 58 - 89 | 32 |
| | Contact | ---- TGIPDRFSGSGSGTDFTLTISRLEPEDFA VYYC (SEQ ID NO. 59) | 57 - 89 | 33 |
| | IMGT | SSRATGIPDRFSGSGSGTDFTLTISRLEPEDFAVYY C (SEQ ID NO. 60) | 53 - 89 | 37 |
| **CDR-L3** | Chothia | QQYGSSLT (SEQ ID NO. 8) | 90 - 97 | 8 |
| | AbM | QQYGSSLT (SEQ ID NO. 8) | 90 - 97 | 8 |
| | Kabat | QQYGSSLT (SEQ ID NO. 8) | 90 - 97 | 8 |
| | Contact | QQYGSSL- (SEQ ID NO. 61) | 90 - 96 | 7 |
| | IMGT | QQYGSSLT (SEQ ID NO. 8) | 90 - 97 | 8 |
| **LFR4** | Chothia | -FGQGTKVEIK (SEQ ID NO. 21) | 98 - 107 | 10 |
| | AbM | -FGQGTKVEIK (SEQ ID NO. 21) | 98 - 107 | 10 |
| | Kabat | -FGQGTKVEIK (SEQ ID NO. 21) | 98 - 107 | 10 |
| | Contact | TFGQGTKVEIK (SEQ ID NO. 62) | 97 - 107 | 11 |
| | IMGT | -FGQGTKVEIK (SEQ ID NO. 21) | 98 - 107 | 10 |

Table 11 below shows CDR sequences in Chothia , AbM, Kabat, Contact and IMGT for VH of Antibody AB4.

**Table 11. Antibody AB4; VH CDR:**

| **Regio n** | **Definitio n** | **Sequence Fragment** | **Residue s** | **Lengt h** |
|---|---|---|---|---|
| **HFR1** | Chothia | QVQLQESGPGLVKPPGTLSLTCAVS----- (SEQ. NO. 32) | 1 - 25 | 25 |
| | AbM | QVQLQESGPGLVKPPGTLSLTCAVS----- (SEQ. NO. 32) | 1 - 25 | 25 |
| | Kabat | QVQLQESGPGLVKPPGTLSLTCAVSGGSIS (SEQ. NO. 14) | 1 - 30 | 30 |
| | Contact | QVQLQESGPGLVKPPGTLSLTCAVSGGSI- (SEQ. NO. 33) | 1 - 29 | 29 |
| | IMGT | QVQLQESGPGLVKPPGTLSLTCAVS----- (SEQ. NO. 32) | 1 - 25 | 25 |
| **CDR-H1** | Chothia | GGSISSSN--- (SEQ. NO. 34) | 26 - 33 | 8 |
| | AbM | GGSISSSNWWS (SEQ. NO. 35) | 26 - 36 | 11 |
| | Kabat | -----SSNWWS (SEQ. NO. 1) | 31 - 36 | 6 |
| | Contact | ----SSSNWWS (SEQ. NO. 36) | 30 - 36 | 7 |
| | IMGT | GGSISSSNW-- (SEQ. NO. 37) | 26 - 34 | 9 |
| **HFR2** | Chothia | WWSWVRQPPGKGLEWIGEI (SEQ. NO.38) | 34 - 52 | 19 |
| | AbM | ---WVRQPPGKGLEWIG-- (SEQ ID NO. 15) | 37 - 50 | 14 |
| | Kabat | ---WVRQPPGKGLEWIG-- (SEQ ID NO. 15) | 37 - 50 | 14 |
| | Contact | --- WVRQPPGKGLE ----- (SEQ. NO.39) | 37 - 47 | 11 |
| | IMGT | -WSWVRQPPGKGLEWIGE- (SEQ. NO.40) | 35 - 51 | 17 |
| **CDR-H2** | Chothia | -----YHSGS--------- (SEQ. NO.41) | 53 - 57 | 5 |
| | AbM | ---EIYHSGSTN------- (SEQ. NO.42) | 51 - 59 | 9 |
| | Kabat | ---EIYHSGSTNYNPSLKS (SEQ ID NO. 2) | 51 - 66 | 16 |
| | Contact | WIGEIYHSGSTN------- (SEQ. NO.43) | 48 - 59 | 12 |
| | IMGT | ----IYHSGST-------- (SEQ. NO.44) | 52 - 58 | 7 |
| **HFR3** | Chothia | TNYNPSLKSRVTISVDKSKNHFSLYVSSVTAADTAVY YCAR (SEQ. NO.63) | 58 - 98 | 41 |
| | AbM | -- YNPSLKSRVTISVDKSKNHFSLYVSSVTAADTAVYYC AR (SEQ. NO.64) | 60 - 98 | 39 |
| | Kabat | ---------RVTISVDKSKNHFSLYVSSVTAADTAVYYCAR (SEQ ID NO. 22) | 67 - 98 | 32 |
| | Contact | -YNPSLKSRVTISVDKSKNHFSLYVSSVTAADTAVYYC -- (SEQ. NO.65) | 60 - 96 | 37 |
| | IMGT | - NYNPSLKSRVTISVDKSKNHFSLYVSSVTAADTAVYY C-- (SEQ. NO.66) | 59-96 | 38 |
| **CDR-H3** | Chothia | --ARLHFDY (SEQ ID NO. 4) | 99 - 105 | 7 |
| | AbM | --ARLHFDY (SEQ ID NO. 4) | 99 - 105 | 7 |
| | Kabat | --ARLHFDY (SEQ ID NO. 4) | 99 - 105 | 7 |
| | Contact | ARARLHFD- (SEQ ID NO. 67) | 97 - 104 | 8 |
| | IMGT | ARARLHFDY (SEQ ID NO. 68) | 97 - 105 | 9 |
| **HFR4** | Chothia | -WGQGTLVTVSS (SEQ ID NO. 23) | 106-116 | 11 |
| | AbM | -WGQGTLVTVSS (SEQ ID NO. 23) | 106-116 | 11 |
| | Kabat | -WGQGTLVTVSS (SEQ ID NO. 23) | 106-116 | 11 |
| | Contact | YWGQGTLVTVSS (SEQ ID NO. 69) | 105-116 | 12 |
| | IMGT | -WGQGTLVTVSS (SEQ ID NO. 23) | 106-116 | 11 |

Table 12 below shows CDR sequences in Chothia , AbM, Kabat, Contact and IMGT for VL of Antibody AB4.

**Table 12. Antibody AB4; VL CDR:**

| **Regio n** | **Definitio n** | **Sequence Fragment** | **Residue s** | **Lengt h** |
|---|---|---|---|---|
| **LFR1** | Chothia | DIVMTQSPLSLPVTPGEPASISC------ (SEQ ID NO. 24) | 1 - 23 | 23 |
| | AbM | DIVMTQSPLSLPVTPGEPASISC------ (SEQ ID NO. 24) | 1 - 23 | 23 |
| | Kabat | DIVMTQSPLSLPVTPGEPASISC------ (SEQ ID NO. 24) | 1 - 23 | 23 |
| | Contact | DIVMTQSPLSLPVTPGEPASISCRSSQSL (SEQ ID NO. 70) | 1 - 29 | 29 |
| | IMGT | DIVMTQSPLSLPVTPGEPASISCRSS --- (SEQ ID NO. 71) | 1 - 26 | 26 |
| **CDR-L1** | Chothia | RSSQSLLHSNGYNYLD-- (SEQ ID NO. 9) | 24 - 39 | 16 |
| | AbM | RSSQSLLHSNGYNYLD-- (SEQ ID NO. 9) | 24 - 39 | 16 |
| | Kabat | RSSQSLLHSNGYNYLD-- (SEQ ID NO. 9) | 24 - 39 | 16 |
| | Contact | ------LHSNGYNYLDWY (SEQ ID NO. 72) | 30 - 41 | 12 |
| | IMGT | ---QSLLHSNGYNY---- (SEQ ID NO. 73) | 27 - 37 | 11 |
| **LFR2** | Chothia | --WYLQKPGQSPQLLIY (SEQ ID NO. 25) | 40 - 54 | 15 |
| | AbM | --WYLQKPGQSPQLLIY (SEQ ID NO. 25) | 40 - 54 | 15 |
| | Kabat | --WYLQKPGQSPQLLIY (SEQ ID NO. 25) | 40 - 54 | 15 |
| | Contact | ----LQKPGQSPQ---- (SEQ ID NO. 7 4) | 42 - 50 | 9 |
| | IMGT | LDWYLQKPGQSPQLLIY (SEQ ID NO. 75) | 38 - 54 | 17 |
| **CDR-L2** | Chothia | ----LGSNRAS (SEQ ID NO. 10) | 55 - 61 | 7 |
| | AbM | ----LGSNRAS (SEQ ID NO. 10) | 55 - 61 | 7 |
| | Kabat | ----LGSNRAS (SEQ ID NO. 10) | 55 - 61 | 7 |
| | Contact | LLIYLGSNRA- (SEQ ID NO. 76) | 51 - 60 | 10 |
| | IMGT | ----LG----- | 55 - 56 | 2 |
| **LFR3** | Chothia | -----GVPOR FSGSGSGTOFTLKISRVEAEOVGVYYC (SEQ ID NO. 26) | 62 - 93 | 32 |
| | AbM | ----- GVPDRFSGSGSGTDFTLKISRVEAEDVGVYYC (SEQ ID NO. 26) | 62 - 93 | 32 |
| | Kabat | -----GVPOR FSGSGSGTOFTLKISRVEAEOVGVYYC (SEQ ID NO. 26) | 62 - 93 | 32 |
| | Contact | ----SGVPORFSGSGSGTOFTLKISRVEAEOVGVYYC (SEQ ID NO. 77) | 61 - 93 | 33 |
| | IMGT | SNRASGVPDRFSGSGSGTDFTLKISRVEAEDVGVY YC (SEQ ID NO. 78) | 57 - 93 | 37 |
| **CDR-L3** | Chothia | MQALQTPPRFT (SEQ ID NO. 11) | 94 - 104 | 11 |
| | AbM | MQALQTPPRFT (SEQ ID NO. 11) | 94 - 104 | 11 |
| | Kabat | MQALQTPPRFT (SEQ ID NO. 11) | 94 - 104 | 11 |
| | Contact | MQALQTPPRF- (SEQ ID NO. 79) | 94 - 103 | 10 |
| | IMGT | MQALQTPPRFT (SEQ ID NO. 11) | 94 - 104 | 11 |
| **LFR4** | Chothia | -FGPGTKVDIK (SEQ ID NO.27) | 105 - 114 | 10 |
| | AbM | -FGPGTKVDIK (SEQ ID NO.27) | 105 - 114 | 10 |
| | Kabat | -FGPGTKVDIK (SEQ ID NO.27) | 105 - 114 | 10 |
| | Contact | TFGPGTKVDIK (SEQ ID NO.80) | 104 - 114 | 11 |
| | IMGT | -FGPGTKVDIK (SEQ ID NO.27) | 105 - 114 | 10 |

Table 13 below shows CDR sequences in Chothia , AbM, Kabat, Contact and IMGT for VH of Antibody AB23.

**Table 13. Antibody AB23; VH CDR:**

| **Regio n** | **Definitio n** | **Sequence Fragment** | **Residue s** | **Lengt h** |
|---|---|---|---|---|
| **HFR1** | Chothia | QVQLQESGPGL VKPPGTLSL TCA VS----- (SEQ. NO. 32) | 1 - 25 | 25 |
| | AbM | QVQLQESGPGLVKPPGTLSLTCAVS----- (SEQ. NO. 32) | 1 - 25 | 25 |
| | Kabat | QVQLQESGPGLVKPPGTLSLTCAVSGGSIS (SEQ ID NO. 14) | 1 - 30 | 30 |
| | Contact | QVQLQESGPGLVKPPGTLSLTCAVSGGSI- (SEQ. NO. 33) | 1 - 29 | 29 |
| | IMGT | QVQLQESGPGLVKPPGTLSLTCAVS----- (SEQ. NO. 32) | 1 - 25 | 25 |
| **CDR-H1** | Chothia | GGSISSSN--- (SEQ. NO.34) | 26 - 33 | 8 |
| | AbM | GGSISSSN\AWS (SEQ. NO.35) | 26 - 36 | 11 |
| | Kabat | -----SSNWWS (SEQ ID NO.1) | 31 - 36 | 6 |
| | Contact | ----SSSNWWS (SEQ. NO.36) | 30 - 36 | 7 |
| | IMGT | GGSISSSNW-- (SEQ. NO.37) | 26 - 34 | 9 |
| **HFR2** | Chothia | WWSWVRQPPGKGLEWIGEI (SEQ. NO.38) | 34 - 52 | 19 |
| | AbM | ---WVRQPPGKGLEWIG-- (SEQ ID NO. 15) | 37 - 50 | 14 |
| | Kabat | ---WVRQPPGKGLEWIG-- (SEQ ID NO. 15) | 37 - 50 | 14 |
| | Contact | --- WVRQPPGKGLE ----- (SEQ. NO.39) | 37 - 47 | 11 |
| | IMGT | -WSWVRQPPGKGLEWIGE- (SEQ. NO.40) | 35 - 51 | 17 |
| **CDR-H2** | Chothia | -----YHSGS--------- (SEQ. NO.41) | 53 - 57 | 5 |
| | AbM | ---EIYHSGSTN------- (SEQ. NO.42) | 51 - 59 | 9 |
| | Kabat | ---EIYHSGSTNYNPSLKS (SEQ ID NO. 2) | 51 - 66 | 16 |
| | Contact | WIGEIYHSGSTN------- (SEQ. NO.43) | 48 - 59 | 12 |
| | IMGT | ----IYHSGST-------- (SEQ. NO.44) | 52 - 58 | 7 |
| **HFR3** | Chothia | TNYNPSLKSRVTISVDTSKNHFYLNLSSVTAADTALY YCAR (SEQ. NO.81) | 58 - 98 | 41 |
| | AbM | -- YNPSLKSRVTISVDTSKNHFYLNLSSVTAADTALYYC AR (SEQ. NO.82) | 60 - 98 | 39 |
| | Kabat | ---------RVTISVDTSKNHFYLNLSSVTAADTALYYCAR (SEQ ID NO. 28) | 67 - 98 | 32 |
| | Contact | -YNPSLKSRVTISVDTSKNHFYLNLSSVTAADTALYYC- - (SEQ. NO.83) | 60 - 96 | 37 |
| | IMGT | - NYNPSLKSRVTISVDTSKNHFYLNLSSVTAADTALYY C-- (SEQ. NO.84) | 59 - 96 | 38 |
| **CDR-H3** | Chothia | --SYFDSGMDV (SEQ ID NO. 5) | 99 - 107 | 9 |
| | AbM | --SYFDSGMDV (SEQ ID NO. 5) | 99 - 107 | 9 |
| | Kabat | --SYFDSGMDV (SEQ ID NO. 5) | 99 - 107 | 9 |
| | Contact | ARSYFDSGMD- (SEQ. NO.85) | 97 - 106 | 10 |
| | IMGT | ARSYFDSGMDV (SEQ. NO.86) | 97 - 107 | 11 |
| **HFR4** | Chothia | -WGQGTTVTVSS (SEQ ID NO. 17) | 108-118 | 11 |
| | AbM | -WGQGTTVTVSS (SEQ ID NO. 17) | 108-118 | 11 |
| | Kabat | -WGQGTTVTVSS (SEQ ID NO. 17) | 108-118 | 11 |
| | Contact | VWGQGTTVTVSS (SEQ ID NO. 51) | 107-118 | 12 |
| | IMGT | -WGQGTTVTVSS (SEQ ID NO. 17) | 108-118 | 11 |

Table 14 below shows CDR sequences in Kabat and IMGT for VL of Antibody AB23.

**Table 14. Antibody AB23; VL CDR:**

| **Region and Definition** | **Sequence Fragment** | **Residues** |
|---|---|---|
| **FR1-IMGT** | EIVMTQSPATLSVSPGERATLSCRAS (SEQ. NO.87) | 1-26 |
| **CDR1-IMGT** | QSVSSN (SEQ. NO.88) | 27-32 |
| **FR2-IMGT** | LAWYQQKLGQASRLLIY (SEQ. NO.89) | 33-49 |
| **CDR2-IMGT** | GA | 50-51 |
| **FR3-IMGT** | SGSGSGTEFTLTISSLQSEDFAVYYC (SEQ. NO.31) | 52-77 |
| **CDR3-IMGT (germline)** | QQYNKWP (SEQ ID NO. 13) | 78-84 |
| | Total | 95 |

| **Region and Definition** | **Sequence Fragment** | **Residues** |
|---|---|---|
| **FR1-Kabat** | EIVMTQSPATLSVSPGERATLSC (SEQ ID NO. 29) | 1-23 |
| **CDR1-Kabat** | RASQSVSSNLA (SEQ ID NO. 12) | 24-34 |
| **FR2-Kabat** | WYQQKLGQASRLLlY (SEQ ID NO. 30) | 35-49 |
| **CDR2-Kabat** | GA----- | 50-51 |
| **FR3-Kabat** | ------ SGSGSGTEFTLTISSLQSEDFAVYYC (SEQ ID NO. 31) | 52-77 |
| **CDR3-Kabat (V gene only)** | QQYNKWP (SEQ ID NO. 13) | 78-84 |
| | Total | 95 |

VH amino acid sequences of exemplified antibodies are reported in the following Table 15.

| **Table 15: VH amino acid sequences of exemplified antibodies** | |
|---|---|
| **Antibody** | **AA of VH** |
| **AB3** | QVQLQESGPGLVKPPGTLSLTCAVSGGSISSSNWWSWVRQPP GKGLEWIGEIYHSGSTNYNPSLKSRVTISVDTSKNQISLQLSSVTA ADTAVYYCARDKMVRGLIDYYYYGLDVWGQGTTVTVSS (SEQ ID NO. 90) |
| **AB4** | QVQLQESGPGLVKPPGTLSLTCAVSGGSISSSNWWSWVRQPP GKGLEWIGEIYHSGSTNYNPSLKSRVTISVDKSKNHFSLYVSSVT AADTAVYYCARARLHFDYWGQGTLVTVSS (SEQ ID NO. 91) |
| **AB23** | QVQLQESGPGLVKPPGTLSLTCAVSGGSISSSNWWSWVRQPP GKGLEWIGEIYHSGSTNYNPSLKSRVTISVDTSKNHFYLNLSSVT AADTALYYCARSYFDSGMDVWGQGTTVTVSS (SEQ ID NO. 92) |

VL amino acid sequences of exemplified antibodies are reported in the following Table 16.

| **Table 16: VL amino acid sequences of exemplified antibodies** | |
|---|---|
| **Antibody** | **AA of VL** |
| **AB3** | EIVLTQSPATLSLSPGERATLSCGASQSVSSSYLAWYQQKPGLA PRLLIYDASSRATGIPDRFSGSGSGTDFTLTISRLEPEDFAVYYCQ QYGSSLTFGQGTKVEIK (SEQ ID NO. 93) |
| **AB4** | DIVMTQSPLSLPVTPGEPASISCRSSQSLLHSNGYNYLDWYLQK PGQSPQLLIYLGSNRASGVPDRFSGSGSGTDFTLKISRVEAEDV GVYYCMQALQTPPRFTFGPGTKVDIK (SEQ ID NO. 94) |
| **AB23** | EIVMTQSPATLSVSPGERATLSCRASQSVSSNLAWYQQKLGQA SRLLIYGASGSGSGTEFTLTISSLQSEDFAVYYCQQYNKWPVTF GQGTKLEIK (SEQ ID NO. 95) |

VH nucleotide sequences of exemplified antibodies are reported in the following Table 17.

| **Table 17: VH nucleotide sequences of exemplified antibodies** | |
|---|---|
| **Antibody** | **DNA of VH** |
| **AB3** | caggtgcagctgcaggagtcgggcccaggactggtgaagcctccggggaccctgtccctc acctgcgctgtctctggtggctccatcagcagtagtaactggtggagttgggtccgccagcc cccagggaaggggctggagtggattggggaaatctatcatagtgggagcaccaactaca acccgtccctcaagagtcgagtcaccatatcagtagacacgtccaagaaccagatctccct gcagctgagctccgtgaccgctgcggacacggccgtgtattactgtgcgagagataagatg gttcggggacttattgactactactactacggtttggacgtctgggggcaagggaccacggt caccgtctcctcag (SEQ ID NO. 96) |
| **AB4** | caggtgcagctgcaggagtcgggcccaggactggtgaagcctccggggaccctgtccctc acctgcgctgtctctggtggctccatcagcagtagtaactggtggagttgggtccgccagcc cccagggaaggggctggagtggattggggaaatctatcatagtgggagcaccaactaca acccgtccctcaagagtcgagtcaccatatcagtagacaagtccaagaaccacttctccct gtacgtgagctctgtgaccgccgcggacacggccgtgtattactgtgcgagagcgagacta cactttgactactggggccaaggaaccctggtcaccgtctcctcag (SEQ ID NO. 97) |
| **AB23** | caggtgcagctgcaggagtcgggcccaggactggtgaagcctccggggaccctgtccctc acctgcgctgtctctggtggctccatcagcagtagtaactggtggagttgggtccgccagcc cccagggaaggggctggagtggattggggaaatctatcatagtgggagcaccaactaca acccgtccctcaagagtcgagtcaccatatcagtagacacgtccaagaaccacttctacct gaacctgagctctgtgaccgctgcggacacggccctgtattactgtgcgagaagctattttga cagcggtatggacgtctgggggcaagggaccacggtcaccgtctcctcag (SEQ ID NO. 98) |

VL nucleotide sequences of exemplified antibodies are reported in the following Table 18.

| **Table 18: VL nucleotide sequences of exemplified antibodies** | |
|---|---|
| **Antibody** | **DNA of VL** |
| **AB3** | gaaattgtgttgacgcagtctccagccaccctgtctttgtctccaggggaaagagccaccctc tcctgcggggccagtcagagtgttagcagcagctacttagcctggtaccagcagaaacctg gcctggcgcccaggctcctcatctatgatgcatccagcagggccactggcatcccagaca ggttcagtggcagtgggtctgggacagacttcactctcaccatcagcagactggagcctga agattttgcagtgtattactgtcagcagtatggtagctccctcacgttcggccaagggaccaa ggtggaaatcaaac (SEQ ID NO. 99) |
| **AB4** | gatattgtgatgactcagtctccactctccctgcccgtcacccctggagagccggcctccatct cctgcaggtctagtcagagcctcctgcatagtaatggatacaactatttggattggtacctgca gaagccagggcagtctccacagctcctgatctatttgggttctaatcgggcctccggggtccc tgacaggttcagtggcagtggatcaggcacagattttacactgaaaatcagcagagtggag gctgaggatgttggggtttattactgcatgcaagctctacaaactcccccgagattcactttcg gccctgggaccaaagtggatatcaaac (SEQ ID NO. 100) |
| **AB23** | gaaatagtgatgacgcagtctccagccaccctgtctgtgtctccaggggaaagagccacc ctctcctgcagggccagtcagagtgttagtagcaacttagcctggtaccagcagaaacttgg ccaggcttccaggctcctcatctatggtgcatcgggcagtgggtctgggacagagttcactct caccatcagcagcctgcagtctgaagattttgcagtttattactgtcagcagtataataagtgg cctgtcacttttggccaggggaccaagttggagatcaaac (SEQ ID NO. 101) |

### EXAMPLES

### METHODS

### NSCLC patients characteristics

Patients with histological diagnosis of advanced (Stage IIIB/IV) NSCLC cancer, who have previously received immune checkpoint inhibitors (ICB) were divided in two cohorts:
Cohort 1. NSCLC patients defined as "Good Responders" following ICB therapy because they are progression free after 10 months of treatment (patients with a OS exceeding 10-month median)
Cohort 2. NSCLC patients defined as "Poor Responders" (or "Fast Progressors") following ICB therapy because they progressed within the first revaluation TC (6-8 weeks), with the appearance of new lesions and rapid decay of the general conditions (loss of at least one point of the ECOG PS)], presence of at least one target measurable lesion according to the Response Evaluation Criteria in Solid Tumors (RECIST 1.1)

### RNA sequencing

RNA was extracted from formalin-fixed paraffin-embedded tissues of 13 patients using AllPrep DNA/RNA FFPE Extraction kit (QIAGEN). Library preparation has been done with the TruSeq RNA Exome kit (Illumina). Raw reads generated from the Illumina NextSeq550 sequencer were demultiplexed with Local Run Manager software.

### RNASeq analysis and antibody design

RNAseq raw data from tumor tissues (from patients represented in Table 19) at the baseline were analyzed for BCR repertoire by MixCR software [9]). At first the software aligns reads against V-, D-, J- and C-gene sequence databases, then assemble overlapping alignments coming from the same molecule covering CD3 regions. In the next step, clonotypes are assembled and the longest possible receptor contig sequences are built. The 5 most abundant VH (2.5%-19%) and VL (1.2%-34%) sequences from 3 patients responding to nivolumab treatment (responders; patients C, 2 and 3) were combined to design complete IgG antibodies in a human-murine chimeric format via Igblast alignment tool [10]). Additionally, Inventors reconstructed 12 antibodies from particularly enriched VH and VL sequences retrieved from 3 fast progressors datasets (patients D, 5 and 8).

**Table 19. Patients cohort used for the study. NA: data not available.**

| **Patient ID** | **Histotype** | **Treatment** | **Chemotherapy after immunotherapy** | **Therapy line** | **Clinical response** | **Number of nivolumab cycles** | **Clinical Class** |
|---|---|---|---|---|---|---|---|
| 2 | Adenocarcinoma | NIVOLUMAB | NA | III | PR | 11 | Good Responder |
| 3 | Adenocarcinoma | NIVOLUMAB | NA | II | CR | 14 | Good Responder |
| 4 | SCC | NIVOLUMAB | No | III | PD | 2 | Fast Progressor |
| 5 | Adenocarcinoma | NIVOLUMAB | No | II | PD | 4 | Fast Progressor |
| 6 | Adenocarcinoma | NIVOLUMAB | No | III | PD | 5 | Fast Progressor |
| 7 | Adenocarcinoma | NIVOLUMAB | NA | II | NA | 4 | Fast Progressor |
| 8 | SCC | NIVOLUMAB | No | II | NA | 7 | Fast Progressor |
| 9 | Adenocarcinoma | NIVOLUMAB | NA | II | PR | 13 | Good Responder |
| 10 | Adenocarcinoma | NIVOLUMAB | NA | II | PD | 6 | Fast Progressor |
| C | NA | NIVOLUMAB | NA | NA | NA | NA | Good Responder |
| D | NA | NIVOLUMAB | NA | NA | NA | NA | Fast Progressor |
| F | NA | NIVOLUMAB | NA | NA | NA | NA | Fast Progressor |

### Antibody production and testing

A total of 42 antibodies were produced transfecting HEK293T cells with mouse-human chimeric heavy chain harboring a specific VH sequence plasmid, paired with a light chain harboring plasmid containing a specific VL retrieved from sequencing (Table 20). As previously described, the 5 most abundant VH and VL sequences from the 3 responders patients (C, 2 and 3) and the 2 most abundant VH and VL from poor responder patients (5, 8 and D) were combined obtaining a total of 42 different antibodies. Antibodies released in the supernatant were purified with AmMagTM Protein A Magnetic Beads and resuspended in pH 7.2 PBS.

Candidate antibodies were tested for their binding capacity on several human lung cancer cell lines: adenocarcinoma cell lines NCI-H1944, NCI-H1437, NCI-H1573 and NCI-H1993; large cell lung cancer cell lines H1581 and H460; squamous cell carcinoma cell line H520 and the human bronchial epithelium immortalized line BEAS-2B. All cell lines were grown in RPMI (Euroclone) medium supplemented with 10% Fetal Bovine Serum (FBS, Gibco), 2mM glutamine and penicillin/streptomycin antibiotics (Gibco). Initial binding screening was performed for 42 candidate antibodies on target lung cancer cells that were fixed with 1% FA in PBS for 10 min. After blocking with 1% BSA/PBS solution for 30 minutes, candidate antibodies were incubated with target cells at the final concentration of 25 ng/uL in 1% BSA/PBS solution. To determine the background level, a mouse IgG2A/k isotype control antibody was used in the same experimental condition. A goat anti-mouse (H+L) conjugated with Alexa Fluor 488 (Invitrogen) was used as a secondary antibody. Binding measurements were assessed by AttuneNxt flow cytometer autosampler (Thermofisher) allowed to process multiple 96-well plates.

**Table 20. Heavy and light (lambda or kappa) variable CDR3 sequences used for antibody reconstruction.**

| **Patient C** | | | | | | | | |
|---|---|---|---|---|---|---|---|---|
| | **VH** | | | | | | | |
| | **Chain** | **Inde x** | **Frequenc y** | **Count** | **CDR3AA** | **V** | **D** | **J** |
| | HC1 | 1 | 19% | 417 | CAKSGRYFDWRRRGEFDY W (SEQ ID NO: 102) | IGHV3-9 | IGHD3-9 | IGHJ4 |
| | HC2 | 2 | 18% | 400 | CARQKTSMLIEHW (SEQ ID NO: 103) | IGHV4-4 | IGHD5-18 | IGHJ4 |
| | HC3 | 3 | 5.3% | 117 | CARDKMVRGLIDYYYYGLDV W (SEQ ID NO: 104) | IGHV4-4 | IGHD3-10 | IGHJ6 |
| | HC4 | 4 | 4.0% | 88 | CARARLHFDYW (SEQ ID NO: 105) | IGHV4-4 | IGHD4-11 | IGHJ4 |
| | HC5 | 6 | 3.6% | 78 | CAKSLMVRGVSDYW (SEQ ID NO: 106) | IGHV3-23 | IGHD3-10 | IGHJ4 |

| | **Lambda VL** | | | | | | | |
|---|---|---|---|---|---|---|---|---|
| | **Chain** | **Inde x** | **Frequenc y** | **Count** | **CDR3AA** | **V** | **D** | **J** |
| | LC1 | 1 | 34% | 578 | CQTWDISTFVVF (SEQ ID NO: 107) | IGLV3-1 | . | IGLJ3 |
| | LC2 | 2 | 14% | 245 | CQAWDSSTAVVF (SEQ ID NO: 108) | IGLV3-1 | . | IGLJ2 |
| | LC3 | 3 | 9.0% | 154 | CSSYSGSINFVF (SEQ ID NO: 109) | IGLV2-34 | . | IGLJ1 |
| | LC4 | 4 | 4.2% | 73 | CYSTDSSGNVF (SEQ ID NO: 110) | IGLV3-10 | . | IGLJ1 |
| | LC5 | 5 | 3.6% | 62 | CAAWDDSLSVLF (SEQ ID NO: 111) | IGLV1-36 | . | IGLJ2 |

| | **Kappa VL** | | | | | | | |
|---|---|---|---|---|---|---|---|---|
| | **Chain** | **Inde x** | **Frequenc y** | **Count** | **CDR3AA** | **V** | **D** | **J** |
| | KC1 | 1 | 27% | 1630 | CQHSSSYPWTF (SEQ ID NO: 112) | IGKV1D-16 | . | IGKJ1 |
| | KC2 | 2 | 7.4% | 453 | CQQYGSSPITF (SEQ ID NO: 113) | IGKV3D-20 | . | IGKJ5 |
| | KC3 | 4 | 3.6% | 221 | CQQYGSSLTF (SEQ ID NO: 114) | IGKV3D-20 | . | IGKJ1 |
| | KC4 | 5 | 3.2% | 198 | CMQALQTPPRFTF (SEQ ID NO: 115) | IGKV2-28 | . | IGKJ3 |
| | KC5 | 6 | 2.9% | 177 | CQQYGSSPAF (SEQ ID NO: 116) | IGKV3D-20 | . | IGKJ1 |

| **Patient 2** | | | | | | | | |
|---|---|---|---|---|---|---|---|---|
| | **VH** | | | | | | | |
| | **Chain** | **Inde x** | **Frequenc y** | **Count** | **CDR3AA** | **V** | **D** | **J** |
| | H21 | 1 | 18% | 169 | CARLPPYPKDFDYW (SEQ ID NO: 117) | IGHV4-30-2 | IGHD3-10 | IGHJ4 |
| | H22 | 2 | 8.8% | 81 | CARHSSSGYYWIDYW (SEQ ID NO: 118) | IGHV4-34 | IGHD3-22 | IGHJ4 |
| | H23 | 3 | 8.0% | 74 | CARLMRSGILTGYYYFDYW (SEQ ID NO: 119) | IGHV4-4 | IGHD3-9 | IGHJ4 |
| | H24 | 4 | 6.2% | 57 | CARLDIAGRSAFDIW (SEQ ID NO: 120) | IGHV4-4 | IGHD6-19 | IGHJ3 |
| | H25 | 5 | 3.9% | 36 | CARIRGGVAFCGGDCEGDY FDYWDYFDYW (SEQ ID NO: 121) | IGHV4-34 | IGHD2-21 | IGHJ4 |

| | **Kappa VL** | | | | | | | |
|---|---|---|---|---|---|---|---|---|
| | **Chain** | **Inde x** | **Frequenc y** | **Count** | **CDR3AA** | **V** | **D** | **J** |
| | K21 | 1 | 4.2% | 359 | CMEALHTPPYTF (SEQ ID NO: 122) | IGKV2-28 | | IGKJ2 |
| | K22 | 4 | 2.4% | 210 | CQQYNSYFMYTF (SEQ ID NO: 123) | IGKV1-5 | . | IGKJ2 |
| | K23 | 5 | 2.4% | 207 | CMQCLHTPPYTF (SEQ ID NO: 124) | IGKV2-28 | . | IGKJ2 |
| | K24 | 6 | 2.4% | 204 | CMEALQTPPFTF (SEQ ID NO: 125) | IGKV2-28 | . | IGKJ2 |
| | K25 | 7 | 1.9% | 163 | CQQYHNLPLTF (SEQ ID NO: 126) | IGKV1-33 | . | IGKJ4 |

| | **Lambda VL** | | | | | | | |
|---|---|---|---|---|---|---|---|---|
| | **Chain** | **Inde x** | **Frequenc y** | **Count** | **CDR3AA** | **V** | **D** | **J** |
| | L21 | 1 | 7.6% | 66 | CATWDDGLEGLVVF (SEQ ID NO: 127) | IGLV1-47 | . | IGLJ3 |
| | L22 | 2 | 6.3% | 55 | CGTWDDSLSVWVF (SEQ ID NO: 128) | IGLV1-44 | . | IGLJ3 |
| | L23 | 3 | 5.4% | 47 | CQSYDNSLSGLWVF (SEQ ID NO: 129) | IGLV1-40 | . | IGLJ3 |
| | L24 | 4 | 5.4% | 47 | CQVWDSSTGVF (SEQ ID NO: 130) | IGLV3-9 | . | IGLJ3 |
| | L25 | 5 | 4.2% | 36 | CSSYAGSNNFVF (SEQ ID NO: 131) | IGLV2-34 | . | IGLJ1 |

| **Patient 3** | | | | | | | | |
|---|---|---|---|---|---|---|---|---|
| | **VH** | | | | | | | |
| | **Chain** | **Index** | **Frequency** | **Count** | **CDR3AA** | **V** | **D** | **J** |
| | H31 | 1 | 13% | 406 | CASCSTVTTFDYW (SEQ ID NO: 132) | IGHV4-34 | IGHD4-17 | IGHJ4 |
| | H32 | 2 | 3.1% | 93 | CAREDYGEGFDAFDIW (SEQ ID NO: 133) | IGHV3-7 | IGHD4-17 | IGHJ3 |
| | H33 | 4 | 2.7% | 83 | CARSYFDSGMDVW (SEQ ID NO: 134) | IGHV4-4 | IGHD3-9 | IGHJ6 |
| | H34 | 5 | 2.5% | 76 | CARAPSYDFWSGYYSQGAF DIW (SEQ ID NO: 135) | IGHV4-55 | IGHD3-3 | IGHJ3 |
| | H35 | 6 | 2.4% | 73 | CARGYGGYVLFYW (SEQ ID NO: 136) | IGHV1-69 | IGHD5-12 | IGHJ4 |

| | **Kappa VL** | | | | | | | |
|---|---|---|---|---|---|---|---|---|
| | **Chain** | **Inde x** | **Frequenc y** | **Count** | **CDR3AA** | **V** | **D** | **J** |
| | K31 | 1 | 2.7% | 789 | CMQALQTPLTF (SEQ ID NO: 137) | IGKV2-28 | . | IGKJ4 |
| | K32 | 2 | 1.7% | 501 | CMQSLQTPPVTF (SEQ ID NO: 138) | IGKV2-28 | . | IGKJ5 |
| | K33 | 3 | 1.5% | 443 | CQQYNKWPVTF (SEQ ID NO: 139) | IGKV3D-15 | . | IGKJ2 |
| | K34 | 4 | 1.2% | 350 | CQQFNNYPITF (SEQ ID NO: 140) | IGKV1D-16 | . | IGKJ5 |
| | K35 | 5 | 1.2% | 350 | CMQGTHWPPITF (SEQ ID NO: 141) | IGKV2-30 | . | IGKJ5 |

| | **Lambda VL** | | | | | | | |
|---|---|---|---|---|---|---|---|---|
| | **Chain** | **Inde x** | **Frequenc y** | **Count** | **CDR3AA** | **V** | **D** | **J** |
| | L31 | 1 | 8.6% | 574 | CSSYGGSNNLVF (SEQ ID NO: 142) | IGLV2-8 | . | IGLJ2 |
| | L32 | 2 | 7.3% | 490 | CQAWDTSTVVF (SEQ ID NO: 143) | IGLV3-1 | . | IGLJ2 |
| | L33 | 3 | 6.3% | 421 | CQAWDSNTYVVF (SEQ ID NO: 144) | IGLV3-1 | . | IGLJ2 |
| | L34 | 4 | 5.2% | 347 | CTSYRSTTVSYVF (SEQ ID NO: 145) | IGLV2-14 | . | IGLJ1 |
| | L35 | 5 | 4.4% | 296 | CCSYAGSYTWVF (SEQ ID NO: 146) | IGLV2-11 | . | IGLJ1 |

| **Patient 5** | | | | | | | | |
|---|---|---|---|---|---|---|---|---|
| | **VH** | | | | | | | |
| | **Chain** | **Inde x** | **Frequenc y** | **Count** | **CDR3AA** | **V** | **D** | **J** |
| | H51 | 1 | 16% | 76 | CAREEGTSVAFDIW (SEQ ID NO: 147) | IGHV4-28 | IGHD1-14 | IGHJ3 |
| | H52 | 2 | 6.0% | 28 | CARERMYFYDTIGYSPFDS W (SEQ ID NO: 148) | IGHV4-34 | IGHD3-22 | IGHJ5 |

| | **Kappa VL** | | | | | | | |
|---|---|---|---|---|---|---|---|---|
| | **Chain** | **Inde x** | **Frequenc y** | **Count** | **CDR3AA** | **V** | **D** | **J** |
| | K51 | 1 | 4.3% | 231 | CQQLHSYPFTF (SEQ ID NO: 149) | IGKV1-9 | . | IGKJ3 |
| | K52 | 2 | 2.8% | 153 | CMQALQTPITF (SEQ ID NO: 150) | IGKV2D-28 | . | IGKJ5 |

| | **Lambda VL** | | | | | | | |
|---|---|---|---|---|---|---|---|---|
| | **Chain** | **Inde x** | **Frequenc y** | **Count** | **CDR3AA** | **V** | **D** | **J** |
| | L51 | 1 | 9.8% | 132 | CSSYGGSNNFVF (SEQ ID NO: 151) | IGLV2-8 | . | IGLJ3 |
| | L52 | 2 | 6.9% | 93 | CAAWDDTLHGVVF (SEQ ID NO: 152) | IGLV1-47 | . | IGLJ2 |

| **Patient D** | | | | | | | | |
|---|---|---|---|---|---|---|---|---|
| | **VH** | | | | | | | |
| | **Chain** | **Inde x** | **Frequenc y** | **Count** | **CDR3AA** | **V** | **D** | **J** |
| | H81 | 1 | 11% | 566 | CAREGAGDGYNPIPRYFDL W (SEQ ID NO: 153) | IGHV4-61 | IGHD5-24 | IGHJ2 |
| | h82 | 2 | 7.4% | 372 | CARAG VLGLTQG VN RAFD I W (SEQ ID NO: 154) | IGHV3-11 | IGHD2-2 | IGHJ3 |

| | **Kappa VL** | | | | | | | |
|---|---|---|---|---|---|---|---|---|
| | **Chain** | **Inde x** | **Frequenc y** | **Count** | **CDR3AA** | **V** | **D** | **J** |
| | K81 | 1 | 16% | 3653 | CQQLNSYPRALTF (SEQ ID NO: 155) | IGKV1-9 | . | IGKJ4 |
| | K82 | 2 | 12% | 2808 | CMQALQTPPTF (SEQ ID NO: 156) | IGKV2D-28 | . | IGKJ2 |

| | **Lambda VL** | | | | | | | |
|---|---|---|---|---|---|---|---|---|
| | **Chain** | **Inde x** | **Frequenc y** | **Count** | **CDR3AA** | **V** | **D** | **J** |
| | L81 | 1 | 17% | 467 | CQSYDSSLSGSNWVF (SEQ ID NO: 157) | IGLV1-40 | . | IGLJ3 |
| | L82 | 2 | 11% | 302 | CQAWDSSTGVVF (SEQ ID NO: 158) | IGLV3-1 | . | IGLJ2 |

### RESULTS

### Example 1

### Identification of NSCLC infiltrating B lymphocyte clones

Retrieval and alignment of antibody sequences from RNASeq data using MixCR software allowed Inventors to identify NSCLC infiltrating B lymphocyte clones. The RNASeq analysis identified 9795 unique immunoglobulin (IgG) variable chains (VH and VL). Five VL chains were common among responder NSCLC patients (responders). However, all were present at very low frequency (<1%) and common among fast progressors. Ten out of 56 (18%) variable chains were present in at least % of responders and were exclusive for responder NSCLC patients although at low frequencies (0,027%-0,59%).

### Example 2

### Design and production of full length antibodies

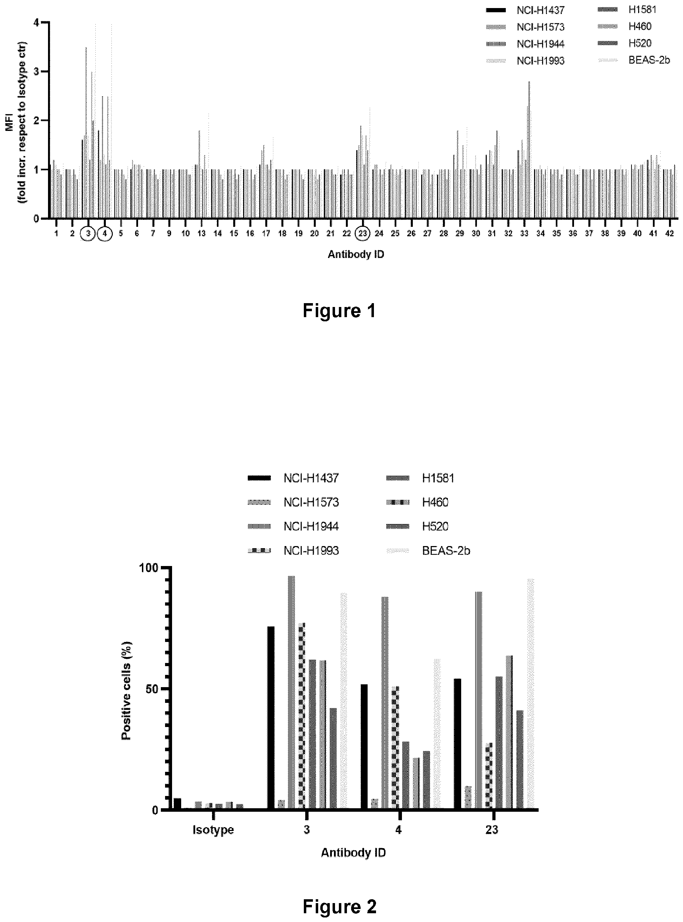

This experimental approach does not allow to identify VH and VL pairs coexpressed in the same cell since they are encoded by different transcripts. However, the i nventors could retrieve the most represented VH and VL (***κ*** and **λ**) chains and rank them for frequency. In order to reconstruct full length antibodies Inventors designed VH and VL pairs based on the most abundant VH coupled to the most abundant ***κ*** and **λ** VL in the same patient. This has allowed the design and effective production of 42 antibodies that were tested for their binding to a panel of 7 human lung cancer cell lines and to human bronchial epithelium immortalized cell line (BEAS-2B) by flow cytometry analysis (Figure 1). As shown in Figure 1, seven of 42 tested antibodies (antibody n. 3, 4, 13, 23, 29, 31 and 33; accounting for 17% of all tested antibodies) were able to efficiently bind to the surface of at least one human lung cancer cell line, and also to BEAS-2B cells, suggesting that tumor targeting antibodies are expressed *in situ* by resident B cells and can potentially bind to bronchial derived human cells as well. Since each VH chain is coupled to 2 different VLs, when the number of VH chains assayed for binding is taken into account, Inventors found that 5 out of 15 VH chains (33%) were able to bind to lung tumor cells in responder NSCLC patients while only 1 out of 6 VH chains (16.5%) among the most represented VH from poor responders. Among those 7 antibodies Inventors further selected and focused further analyses on antibodies n. AB3, AB4 and AB23 only. Their amino acid sequences and nucleotide sequences encoding thereof are reported in Tables 1-18 above.

Figure 2 indicates the percentage of cells positive for selected antibodies. These data suggest that antibodies n. AB3, AB4, AB23 can bind to a wide panel of different lung tumor types (adenocarcinoma as shown by NCI-H1944, NCI-H1437, NCI-H1573 and NCI-H1993 cells binding; large cell lung cancer as shown by H1581 and H460 cells binding; squamous cell carcinoma as shown by H520 cells binding).

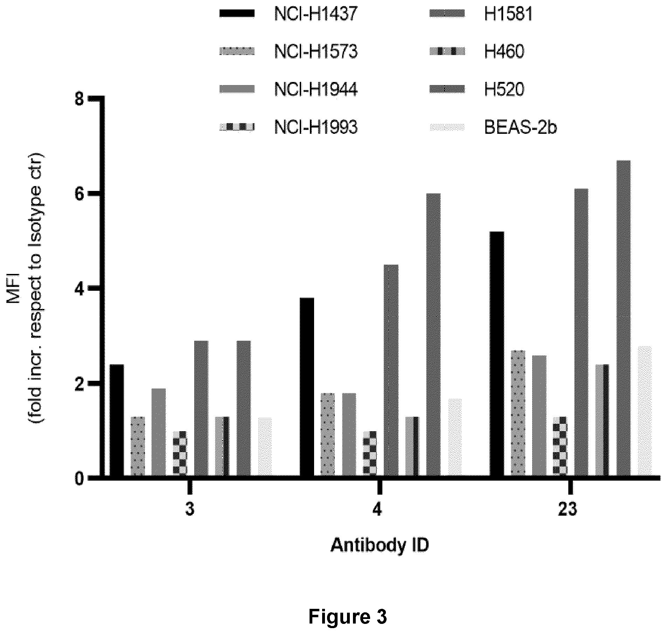

Selected antibodies were also tested on the same cell lines previously fixed with 1% Formaldehyde (FA). As shown in Figure 3, binding properties are not compromised by cell fixation procedure.

### Conclusions

Herein Inventors demonstrated the possibility to screen in situ NSCLC tumor infiltrated B cells for expressing tumor targeting antibodies starting from RNASeq data. The inventors observed a higher percentage of tumor targeting antibodies from ICI (nivolumab) NSCLC responders (33%) as compared to poor responders (17%). Using this approach Inventors have identified 3 fully human antibodies (n. AB3, AB4 and AB23) that can potentially be used in immunotherapy-based strategies that target lung cancer (i.e. antibody-drug conjugates (ADC), CAR harboring cells, nude antibody for antibody-dependent cellular cytotoxicity (ADCC) etc.). These biomolecules present several advantages: they have been already produced in humans, thus being compatible with the human immune response and they are likely contributing to ICI induced clinical response in NSCLC patients.

### BIBLIOGRAPHY

1.Dieu-Nosjean, M.-C.; Giraldo, N.A.; Kaplon, H.; Germain, C.; Fridman, W.H.; Sautès-Fridman, C. Tertiary lymphoid structures, drivers of the anti-tumor responses in human cancers. Immunol. Rev. 2016, 271, 260-275.
2. Colbeck, E.J.; Ager, A.; Gallimore, A.; Jones, G.W. Tertiary Lymphoid Structures in Cancer: Drivers of Antitumor Immunity, Immunosuppression, or Bystander Sentinels in Disease? Front. Immunol. 2017, 8, 1830.
3. Sautès-Fridman, C.; Lawand, M.; Giraldo, N.A.; Kaplon, H.; Germain, C.; Fridman, W.H.; Dieu-Nosjean, M.-C. Tertiary Lymphoid Structures in Cancers: Prognostic Value, Regulation, and Manipulation for Therapeutic Intervention. Front. Immunol. 2016, 7, 407.
4. Martinet, L.; Garrido, I.; Filleron, T.; Le Guellec, S.; Bellard, E.; Fournie, J.J.; Rochaix, P.; Girard, J.P. Human Solid Tumors Contain High Endothelial Venules: Association with T- and B-Lymphocyte Infiltration and Favorable Prognosis in Breast Cancer. Cancer Res. 2011, 71, 5678-5687.
5. Germain, C.; Gnjatic, S.; Tamzalit, F.; Knockaert, S.; Remark, R.; Goc, J.; Lepelley, A.; Becht, E.; Katsahian, S.; Bizouard, G.; et al. Presence of B Cells in Tertiary Lymphoid Structures Is Associated with a Protective Immunity in Patients with Lung Cancer. Am. J. Respir. Crit. Care Med. 2014, 189, 832-844.
6. Dieu-Nosjean, M.-C.; Antoine, M.; Danel, C.; Heudes, D.; Wislez, M.; Poulot, V.; Rabbe, N.; Laurans, L.; Tartour, E.; de Chaisemartin, L.; et al. Long-Term Survival for Patients With Non-Small-Cell Lung Cancer With Intratumoral Lymphoid Structures. JCO 2008, 26, 4410-4417.
7. Silina, K.; Soltermann, A.; Attar, F.M.; Casanova, R.; Uckeley, Z.M.; Thut, H.; Wandres, M.; Isajevs, S.; Cheng, P.; Curioni-Fontecedro, A.; et al. Germinal Centers Determine the Prognostic Relevance of Tertiary Lymphoid Structures and Are Impaired by Corticosteroids in Lung Squamous Cell Carcinoma. Cancer Res. 2018, 78, 1308-1320.
8. Nicolini F, Bocchini M, Angeli D, Bronte G, Delmonte A, Crinò L, Mazza M. Fully Human Antibodies for Malignant Pleural Mesothelioma Targeting. Cancers (Basel). 2020 Apr 8;12(4):915. doi: 10.3390/cancers12040915. PMID: 32276524; PMCID: PMC7226231.
9. Bolotin DA, Poslavsky S, Mitrophanov I, Shugay M, Mamedov IZ, Putintseva EV, Chudakov DM. MiXCR: software for comprehensive adaptive immunity profiling. Nat Methods. 2015 May;12(5)-380-1. doi: 10.1038/nmeth.3364. PMID: 25924071.
10. https://www.ncbi.nlm.nih.gov/igblast/

## Claims

1. An isolated antibody or antigen binding fragment thereof comprising:
a. a heavy chain variable domain (VH) comprising:
i. a CDR1 sequence of an amino acid sequence of: SEQ ID NO.1;
ii. a CDR2 sequence of an amino acid sequence of SEQ ID NO. 2; and
iii. a CDR3 sequence of an amino acid sequence selected from the group consisting of SEQ ID NO. 4, 3 and 5 and/or
b. a light chain variable domain (VL) comprising:
i. a CDR1 sequence of an amino acid sequence selected from the group consisting of: SEQ ID NO. 9, 6 and 12;
ii. a CDR2 sequence of an amino acid sequence selected from the group consisting of: SEQ ID NO. 10, 7 and 13; and
iii. a CDR3 sequence of an amino acid sequence selected from the group consisting of: SEQ ID NO. 11, 8 and 14.

2. The antibody or antigen binding fragment thereof according to claim 1 comprising as CDRs:
SEQ ID NO: 1 and SEQ ID NO: 2 and SEQ ID NO: 4 and SEQ ID NO: 9 and SEQ ID NO: 10 and SEQ ID NO: 11 or respective Kabat, IMGT, Chothia, AbM, or Contact CDRs; or
SEQ ID NO: 1 and SEQ ID NO: 2 and SEQ ID NO: 3 and SEQ ID NO: 6 and SEQ ID NO: 7 and SEQ ID NO: 8 or respective Kabat, IMGT, Chothia, AbM, or Contact CDRs; or
SEQ ID NO: 1 and SEQ ID NO: 2 and SEQ ID NO: 5 and SEQ ID NO: 12 and SEQ ID NO: 13 and SEQ ID NO: 14 or respective Kabat, IMGT, Chothia, AbM, or Contact CDRs.

3. The antibody or antigen binding fragment thereof according to claim 1 or 2 comprising:
a. a heavy chain variable domain sequence of an amino acid sequence selected from the group consisting of: SEQ ID NO: 91, SEQ ID NO: 90 and SEQ ID NO:92 or
b. a light chain variable domain sequence of an amino acid sequence selected from the group consisting of: SEQ ID NO: 94, SEQ ID NO: 93 and SEQ ID NO:95, or
c. the heavy chain variable domain of (a) and the light chain variable domain of (b).

4. The antibody or antigen binding fragment thereof according to claim 3 wherein the isolated antibody is AB4 comprising a heavy chain comprising or consisting of a sequence of SEQ ID NO: 91 and a light chain comprising or consisting of a sequence of SEQ ID NO: 94; or AB3 comprising a heavy chain comprising or consisting of a sequence of SEQ ID NO: 90 and a light chain comprising or consisting of a sequence of SEQ ID NO:93; or AB23 comprising a heavy chain comprising or consisting of a sequence of SEQ ID NO:92 and a light chain comprising or consisting of a sequence of SEQ ID NO:95.

5. The antibody or antigen binding fragment thereof according to anyone of claims 1-4 comprising a heavy chain encoded by a nucleotide sequence selected from the group consisting of: SEQ ID NO: 97, SEQ ID NO: 96 and SEQ ID NO: 98 and a light chain encoded by a nucleotide sequence selected from the group consisting of: SEQ ID NO: 100, SEQ ID NO: 99 and SEQ ID NO.101.

6. The isolated antibody or antigen binding fragment thereof according to any one of previous claims being an IgG1 or IgG2 or IgG3 or IgG4 antibody, preferably an IgG1 kappa antibody, an IgG1 lambda antibody, an IgG4 kappa antibody or an IgG4 lambda antibody, preferably said IgG1 or IgG2 or IgG3 or IgG4 is human IgG1 or human IgG2 or human IgG3 or human IgG4.

7. An isolated polynucleotide comprising at least one sequence that encodes the antibody or antigen binding fragment thereof according to any one of previous claims, preferably said polynucleotide being a cDNA.

8. A vector comprising the polynucleotide according to claim 7 preferably said vector being selected from the group consisting of a plasmid, a viral vector, a non-episomal mammalian vector, an expression vector and a recombinant expression vector.

9. An isolated cell comprising the polynucleotide according to claim 7 or the vector according to claim 8, preferably said isolated cell being a hybridoma or a Chinese Hamster Ovary (CHO) cell or a Human Embryonic Kidney cell (HEK293).

10. The antibody or antigen binding fragment thereof according to any one of claims 1-6 or the polynucleotide according to claim 7 or the vector according to claim 8 or the cell according to claim 9 for use as a medicament.

11. The antibody or antigen binding fragment thereof according to any one of claims 1-6 or the polynucleotide according to claim 7 or the vector according to claim 8 or the cell according to claim 9 for use in the treatment of lung cancer, preferably non-small-cell lung cancer (NSCLC).

12. A pharmaceutical composition comprising the isolated antibody or antigen binding fragment thereof according to any one of claims 1 to 6 or the polynucleotide according to claim 7 or the vector according to claim 8 or the cell according to claim 9 and at least one pharmaceutically acceptable carrier, preferably for use in the treatment of lung cancer, preferably non-small-cell lung cancer (NSCLC).

13. A chimeric antigen receptor (CAR) comprising an antigen-binding fragment of the antibody of anyone of claims 1 to 6 linked to an intracellular domain of a T cell, said intracellular domain preferably comprising one or more signaling domains.

14. An antibody-drug conjugate (ADC) comprising the antibody of anyone of claims 1-6 or an antigen binding fragment thereof conjugated with a cytotoxic drug.

15. The antibody of anyone of claims 1-6 for use for inducing antibody-dependent cellular cytotoxicity (ADCC) and/or complement dependent cytotoxicity (CDC) in a subject affected by a cancer, preferably lung cancer, in particular NSCLC, or for use in an adoptive cell therapy for the treatment of lung cancer.
